# EUROPEAN PATENT SPECIFICATION

(11) **EP 1 448 551 B1**
(45) Date of publication and mention of the grant of the patent: **31.10.2007**
(21) Application number: 02772689.2
(22) Date of filing: 03.10.2002
(51) Int. Cl.: C07D 401/12, C07F 5/02, C07C 235/04

(54) **PROCESSES FOR THE PREPARATION OF SUBSTITUTED BICYCLIC DERIVATIVES FOR THE TREATMENT OF ABNORMAL CELL GROWTH**
VERFAHREN FÜR DIE HERSTELLUNG SUBSTITUIERTER BICYCLISCHER DERIVATE ZUR BEHANDLUNG VON ANOMALEM ZELLWACHSTUM
PROCEDES DE PREPARATION DE DERIVES BICYCLIQUES SUBSTITUES UTILISE POUR TRAITER LA CROISSANCE CELLULAIRE ANORMALE

(30) Priority: 30.11.2001 US 334647 P
(43) Date of publication of application: 25.08.2004
(73) Proprietor: OSI Pharmaceuticals, Inc., Melville, NY 11747 (US); Pfizer, Inc., New York, NY 10017-5755 (US)
(72) Inventor: Ripin, David H. B. Pfizer Global Research Develop, Groton, CT 06340 (US)
(74) Representative: Blakey, Alison Jane
(86) International application number: PCT/IB2002/004097
(87) International publication number: WO 2003/045939

(56) References cited:
- WO-A-01/98277
- WO-A-97/30034

## Description

### Cross-Reference to Related Application

This application claims the benefit of U.S. Provisional Patent Application No. 60/334,647, filed November 30,2001.

### Background of the Invention

This invention relates to processes for the preparation compounds of formula 1 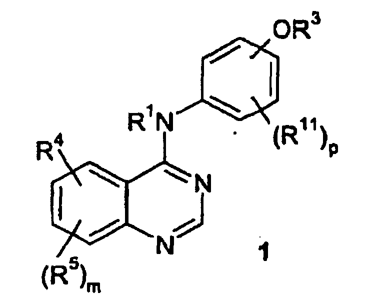 and to pharmaceutically acceptable salts, solvates and prodrugs thereof wherein R¹, R³, R⁴, R⁵, R¹¹, m and p are as defined herein.

The compounds of formula 1 are useful in the treatment of abnormal cell growth, such as cancer, in mammals, are described in U. S. Patent Application No. 09/883,752, filed June 18, 2001.

It is known that a cell may become cancerous by virtue of the transformation of a portion of its DNA into an oncogene (i.e., a gene which, on activation, leads to the formation of malignant tumor cells). Many oncogenes encode proteins that are aberrant tyrosine kinases capable of causing cell transformation. Alternatively, the overexpression of a normal proto-oncogenic tyrosine kinase may also result in proliferative disorders, sometimes resulting in a malignant phenotype.

Receptor tyrosine kinases are enzymes which span the cell membrane and possess an extracellular binding domain for growth factors such as epidermal growth factor, a transmembrane domain, and an intracellular portion which functions as a kinase to phosphorylate specific tyrosine residues in proteins and hence to influence cell proliferation. Other receptor tyrosine kinases include c-erbB-2, c-met, tle-2. PDGFr, FGFr, and VEGFR. It is known that such kinases are frequently aberrantly expressed in common human cancers such as breast cancer, gastrointestinal cancer such as colon, rectal or stomach cancer, leukemia, and ovarian, bronchial or pancreatic cancer. It has also been shown that epidermal growth factor receptor (EGFR), which possesses tyrosine kinase activity, is mutated and/or overexpressed in many human cancers such as brain, lung, squamous cell, bladder, gastric, breast, head and neck, oesophageal, gynecological and thyroid tumors.

Accordingly, it has been recognized that inhibitors of receptor tyrosine kinases are useful as selective inhibitors of the growth of mammalian cancer cells. For example, erbstatin, a tyrosine kinase inhibitor, selectively attenuates the growth in athymic nude mice of a transplanted human mammary carcinoma which expresses epidermal growth factor receptor tyrosine kinase (EGFR) but is without effect on the growth of another carcinoma which does not express the EGF receptor. Thus, the compounds of the present invention, which are selective inhibitors of certain receptor tyrosine kinases, are useful in the treatment of abnormal cell growth, in particular cancer, in mammals. In addition to receptor tyrosine kianses, the compounds of the present invention can also display inhibitory activity against a variety of other non-receptor tyrosine kinases (eg: Ick, src, abl) or serine/threonine kinases (e.g.: cyclin dependent kinases).

Various other compounds, such as styrene derivatives, have also been shown to possess tyrosine kinase inhibitory properties. Five European patent publications, namely EP 0 566 226 A1 (published October 20, 1993), EP 0 602 851 A1 (published June 22, 1994), EP 0 635 507 A1 (published January 25, 1995), EP 0 635 498 A1 (published January 25, 1995), and EP 0 520 722 A1 (published December 30, 1992), refer to certain bicyclic derivatives, in particular quinazoline derivatives, as possessing anti-cancer properties that result from their tyrosine kinase inhibitory properties. Also, World Patent Application WO 92/20642 (published November 26, 1992), refers to certain bis-mono and bicyclic aryl and heteroaryl compounds as tyrosine kinase inhibitors that are useful in inhibiting abnormal cell proliferation. World Patent Applications WO96/16960 (published June 6, 1996), WO 96/09294 (published March 6, 1996), WO 97/30034 (published August 21, 1997), WO 98/02434 (published January 22, 1998), WO 98/02437 (published January 22, 1998), and WO 98/02438 (published January 22, 1998), also refer to substituted bicyclic heteroaromatic derivatives as tyrosine kinase inhibitors that are useful for the same purpose. Other patents that refer to anti-cancer compounds are United States patent numbers US6284764 (filed January 20, 2000) and US6465449 (filed January 20, 2000).

### Summary of the Invention

The present invention relates to a process for preparing a compound of formula 1 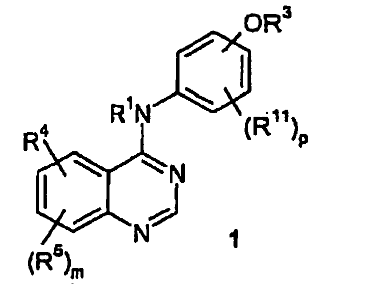 pharmaceutically acceptable salts, and solvates thereof, wherein:
m is an integer from 0 to 3;
p is an integer from 0 to 4;
each R¹ and R² is independently selected from H and C₁-C₈ alkyl;
R³ is -(CR¹R²)ₜ(4 to 10 membered haterocyclic), wherein t is an integer from 0 to 5, said heterocyclic group is optionally fused to a benzene ring or a C₅-C₈ cycloalkyl group, the -(CR¹R²)ₜ- moiety of the foregoing R³ group optionally includes a carbon-carbon double or triple bond where t is an integer between 2 and 5, and the foregoing R⁸ groups, including any optional fused rings referred to above, are optionally substituted by 1 to 5 R⁸ groups;
R⁴ is -C≡C-(CR¹⁰R¹⁷)ₜR⁹, -C=C-(CR¹⁶R¹⁷)ₜ-R⁹, -C≡C-(CR¹⁸R¹⁷)ₖR¹³, or -C=C-(CR¹⁶R¹⁷)ₖR¹³, wherein the attachment point to R⁸ is through a carbon atom of the R⁹ group, each k is an integer from 1 to 3, each t is an integer from 0 to 5, and each m is an integer from 0 to 3;
each R⁵ is independently selected from halo, hydroxy, -NR¹R², C₁-C₆ alkyl, trifluoromethyl, C₁-C₆ alkoxy, trifluoromethoxy, -NR⁶C(O)R¹, -C(O)NR⁶R⁷, -SO₂NR⁶R⁷, -NR⁶C(O)NR⁷R¹, and -NR⁶C(O)OR⁷;
each R⁵, R^{6a} and R⁷ is independently selected from H, C₁-C₆ alkyl, -(CR¹R²)ₜ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R⁶ and R⁷ groups are optionally substituted with 1 to 3 substituents independently selected from halo, cyano, nitro, -NR¹R², trifluoromethyl, trifluoromethoxy, C₁-C₆ alkyl, C₂-C₆ alkanyl, C₂-C₆ alkynyl, hydroxy, and C₁-C₆ alkoxy,
or R⁶ and R⁷, or R^{6a} and R⁷, when attached to a nitrogen atom (including the same nitrogen n atom or two separate nitrogen atoms in proximity to each other through interconnection by, for instance, -C(O) or -SO₂-), can be taken together to form a 4 to 10 membered heterocyclic ring which may include 1 to 3 additional hetero moieties, in addition to the nitrogen to which said R⁶, R^{6a}, and R⁷ are attached, selected from N, N(R¹), O, and S, provided two O atoms, two S atoms or an O and S atom are not attached directly to each other;
each R⁸ is independently selected from oxo (=O), halo, cyano, nitro, trifluoromethoxy, trifluoromethyl, azido, hydroxy, C₁-C₆ alkoxy, C₁-C₁₀ alkyl, C₂-C₆ alkenyl, C₂-C₆ alkynyl, -C(O)R⁶, -C(O)OR⁶, -OC(O)R⁶, -NR⁶C(O)R⁷, -NR⁶SO₂NR⁷R¹, -NR⁶C(O)NR¹R⁷, -NR⁶C(O)OR⁷, -C(O)NR^{O}R⁷, -NR⁶R⁷, -NR⁶OR⁷, -SO₂NR⁶R⁷, -S(O)ⱼ(C₁-C₆ alkyl) wherein j is an integer from 0 to 2, -(CR¹R²)ₜ(C₆-C₁₀ aryl), -(CR¹R²)ₜ(4 to 10 membered heterocyclic), -(CR¹R²)_{q}C(O)(CR¹R²)ₜ(C₆-C₁₀ aryl), -(CR¹R²)_{q}C(O)(CR¹R²)ₜ(4 to 10 membered heterocyclic), -(CR¹R²)ₜO(CR¹R²)_{q}(C₆-C₁₀ aryl), -(CR¹R²)ₜO(CR¹R²)_{q}(4 to 10 membered heterocyclic), -(CR¹R²)_{q}S(O)ⱼ(CR¹R²)ₗ(C₈-C₁₀ aryl), and -(CR¹R²)_{q}S(O)ⱼ(CR¹R²)ₜ(4 to 10 membered heterocyclic), wherein j is 0, 1 or 2, q and t are each independently an integer from 0 to 5, 1 or 2 ring carbon atoms of the heterocyclic moieties of the foregoing R⁸ groups are optionally substituted with an oxo (=O) moiety, and the alkyl, alkenyl, alkynyl, aryl and heterocyclic moieties of the foregoing R⁸ groups are optionally substituted with 1 to 3 substituents independently selected from halo, cyano, nitro, trifluoromethyl, trifluoromethoxy, azido, -OR⁶, -C(O)R⁶, -C(O)OR⁶, -OC(O)R⁶, -NR⁶C(O)R⁷, -C(O)NR⁶R⁷, -NR⁶R⁷, -NR⁶OR⁷, C₁-C₆ alkyl, C₂-Ca alkenyl, C₂-C₆ alkynyl, -(CR¹R²)ₜ(C₆-C₁₀ aryl), and -(CR¹R²)ₜ(4 to 10 membered heterocyclic), wherein t is an integer from 0 to 5;
R⁹ is a non-aromatic mono-cyclic ring, a fused or bridged bicyclic ring, or a spirocyclic ring, wherein said ring contains from 3 to 12 carbon atoms in which from 0 to 3 carbon atoms are optionally replaced with a hetero moiety independently selected from N, O, S(O)ⱼ wherein j is an integer from 0 to 2, and -NR¹-, provided that two O atoms, two S(O)ⱼ moieties, an O atom and a S(O)ⱼ moiety, an N atom and an S atom, or an N atom and an O atom are not attached directly to each other within said ring, and wherein the carbon atoms of said ring are optionally substituted with 1 or 2 R⁸ groups;
each R¹¹ is independently selected from the substituents provided in the definition of R⁸, except R¹¹ is not oxo (=O);
R¹² is R⁶, -OR⁶, -OC(O)R⁶, -OC(O)NR⁶R⁷, -OCO₂R⁶, -S(O)ⱼR⁶, -S(O)ⱼNR⁶R⁷, -NR⁶R⁷, -NR⁶C(O)R⁷, -NR⁶SO₂R⁷, -NR⁶C(O)NR^{6a}R⁷, -NR⁶SO₂NR^{6a}R⁷, -NR⁶CO₂R⁷, CN, -C(O)R⁶, or halo, wherein is an integer from 0 to 2;
R¹³ is -NR¹R¹⁴ or -OR¹⁴;
R¹⁴ is H, R¹⁵, -C(O)R¹⁵, -SO₂R¹⁵, -C(O)NR¹⁵R⁷, -SO₂NR¹⁵R⁷, or-CO₂R¹⁵;
R¹⁵ is R¹⁸, -(CR¹R²)ₜ(C₈-C₁₀ aryl), -(CR¹R²)ₜ(4 to 10 membered heterocyclic), wherein t is an integer from 0 to 5, 1 or 2 ring carbon atoms of the heterocyclic group are optionally substituted with an oxo (=O) moiety, and the aryl and heterocyclic moieties of the foregoing R¹⁹ groups are optionally substituted with 1 to 3 R⁸ substituents;
each R¹⁶ and R¹⁷ is independently selected from H, C₁-C₆ alkyl, and -CH₂OH, or R¹⁶ and R¹⁷ are taken together as -CH₂CH₂- or -CH₂CH₂CH₂-;
R¹⁸ is C₁-C₆ alkyl wherein each carbon not bound to a N or O atom, or to S(O)ⱼ, wherein is an integer from 0 to 2, is optionally substituted with R¹²;
and wherein any of the above-mentioned substituents comprising a CH₃ (methyl), CH₂ (methylene), or CH (methine) group, which is not attached to a halogeno, SO or SO₂ group or to a N, O or S atom, is optionally subsituted with a group selected from hydroxy, halo, C₁-C₄ alkyl, C₁-C₄ alkoxy and -NR¹R², which comprises reacting a compound of formula 2 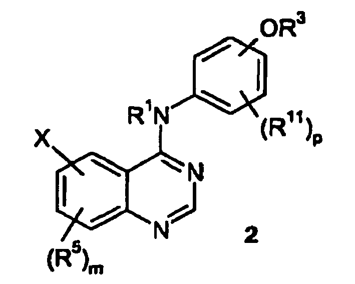 wherein X is a halide and R¹, R³, R⁵, R¹¹, m and p are as defined for formula 1 with a compound of formula H-C≡C-(CR¹⁶R¹⁷)ₜR⁸, M-C=C(CR¹⁶R¹⁷)ₜ-R⁸, H-C≡C-(CR¹⁶R¹⁷)ₖR¹³, or M-C=C-(CR¹⁶R¹⁷)ₖR¹³, wherein the attachment point to R⁸, is through a carbon atom of the R⁹ group, each k is an integer from 1 to 3, each t is an integer from 0 to 5, and each m is an integer from 0 to 3, wherein M is selected from the group consisting of H. B(R¹⁹)₂, Al(R²⁰)₂, Sn(R²¹)₃, MgW. or ZnW, wherein R¹⁹ is selected from the group consisting of 9-BBN, C₁-C₁₀ alkyl, C₁-C₁₀ alkoxy, C₃-C₁₀ cycloalkyl, and halo, wherein R²⁰ is selected from the group consisting of C₁-C₁₀ alkyl, C₁-C₁₀ alkoxy, C₃-C₁₀ cycloalkyl, and helo, wherein R²¹ is C₁-C₁₀ alkyl and wherein W is Cl. Br or I, wherein said reaction is carried out in the presence of a palladium catalyst, a ligand, a base, and an optional additive.

In a specific embodiment of the present invention the process of the present invention is used to make compounds of formula 1 wherein R³ is -(CR¹R²)ₜ(4 to 10 membered heterocyclic), wherein t is an integer from 0 to 5, and the foregoing R³ groups are optionally substituted by 1 to 3 R⁵ groups; said heterocyclic group is optionally fused to a benzene ring or a C₅-C₈ cycloalkyl group, and the foregoing R³ groups, including any optional fused rings referred to above, are optionally substituted by 1 to 3 R⁸ groups.

Other specific embodiments of the present invention the process of the present invention is used to make compounds of formula 1, wherein R³ is selected from 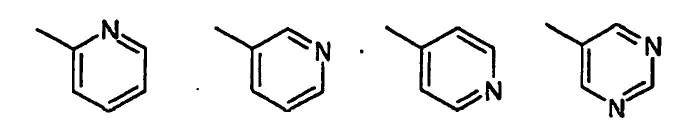 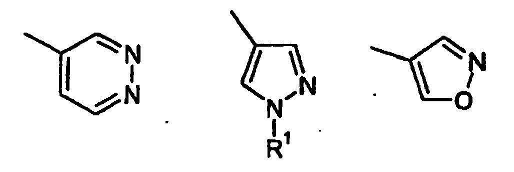 and 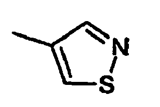 wherein the foregoing R³ groups are optionally substituted by 1 to 3 R⁸ groups.

Other specific embodiments the process of the present invention is used to make compounds of formula 1 wherein R³ is pyridin-3-yl optionally substituted by 1 to 3 R⁸ groups.

Other specific embodiments the process of the present invention is used to make compounds of formula 1, wherein R⁴ is -C≡C-(CR¹⁶R¹⁷)ₜR⁸, wherein m is an integer from 0 to 3. and t is an integer from 0 to 5.

Other specific embodiments the process of the present invention is used to make compounds of formula 1, wherein R⁴ is C≡C-(CR¹⁸R¹⁷)ₜR⁹, wherein m is an integer from 0 to 3, and t is an integer from 0 to 5, wherein R⁸ is selected from 3-piperidinyl and 4-piperidinyl each of which is optionally substituted with 1 or 2 R⁸ groups.

Other specific embodiments the process of the present invention is used to make compounds of formula 1, wherein R⁴ is -C=C-(CR¹⁸R¹⁷)ₗ-R⁹, wherein m is an integer from 0 to 3. and t is an integer from 0 to 5.

Other specific embodiments the process of the present invention is used to make compounds of formula 1, wherein R⁴ is -C=C-(CR¹⁶R¹⁷)ₜ-R⁹, wherein m is an integer from 0 to 3, and t is an integer from 0 to 5, wherein R⁹ is selected from 3-piperidinyl and 4-piperidinyl (optionally substituted with 1 or 2 R⁸ groups).

Other specific embodiments the process of the present invention is used to make compounds of formula 1, wherein R⁴ is -C≡C-(CR¹⁸R¹⁷)ₖR¹³, wherein k is an integer from 1 to 3 and m is an integer from 0 to 3.

Other specific embodiments the process of the present invention is used to make compounds of formula 1. wherein R⁴ is -C≡C-(CR¹⁸R¹⁷)ₖR¹³, wherein k is an integer from 1 to 3 and m is an integer from 0 to 3, wherein R¹³ is -NR¹R¹⁴, wherein R¹⁴ is selected from -C(O)R¹⁵, -SO₂R¹⁵, and -C(O)NR¹⁵R⁷.

Other specific embodiments the process of the present invention is used to make compounds of formula 1, wherein R⁴ is -C=C-(CR¹⁸R¹⁷)ₖR¹³, wherein k is an integer from 1 to 3 and m is an integer from 0 to 3.

Other specific embodiments the process of the present invention is used to make compounds of formula 1, wherein R⁴ is -C=C-(CR¹⁶R¹⁷)ₖR¹³, wherein k is an integer from 1 to 3 and m is an integer from 0 to 3, wherein R¹³ is -NR¹R¹⁴, wherein R¹⁴ is selected from -C(O)R¹⁵, -SO₂R¹⁶, and -C(O)NR¹⁶R⁷.

Other specific embodiments of the process of the present invention is used to make compounds of formula 1, wherein R⁴ is -C≡C-(CR¹⁸R¹⁷)ₖR¹³ or -C=C-(CR¹⁸¹⁷)ₖR¹³, wherein k is an integer from 1 to 3 and m is an integer from 0 to 3, R¹³ is -NR¹R¹⁴ or -OR¹⁴, R¹⁴ is R¹⁵, R¹⁵ is R¹⁸ and R¹⁸ is C₁-C₈ alkyl optionally substituted by -OR⁶, -S(O)ⱼR⁸, -NR⁸R⁷, -NR⁸C(O)R⁷, -NR⁶SO₂R⁷, -NR⁶CO₂R⁷, CN, -C(O)R⁶, or halo.

Specific preferred compounds prepared using the process of the present invention include those selected from the group consisting of:
(±)-[3-Methyl-4-(pyridin-3-yloxy)-phenyl]-(6-piperidin-3-ylethynyl-quinazolin-4-yl)-amine;
2-Methoxy-N-(3-{4-[3-methyl-4-(pyridin-3-yloxy)-phenylamino]-quinazalin-6-yl}-prop-2-ynyl)-acetamide
(±)-[3-Methyl-4-(6-methyl-pyridin-3-yloxy)-phenyl]-(6-piperidin-3-ylethynyl-quinazolin-4-yl)-amine;
[3-Methyl-4-(6-methyl-pyridin-3-yloxy)-phenyl]-(6-piperidin-4-ylethynyl-quinazolin-4-yl)-amine;
2-Methoxy-N-(3-{4-[3-methyl-4-(6-methyl-pyridin-3-yloxy)-phenylamino]-quinazolin-6-yl}-prop-2-ynyl)-catamide;
2-Fluoro-N-(3-{4-[3-methyl-4-(6-methyl-pyridin-3-yloxy)-phenylamino]-quinazolin-6-yl}-prop-2-ynyl)-acetamide;
E-2-Methoxy-N-(3-{4-[3-methyl-4-(6-methyl-pyridin-3-yloxy)-phenylamino]-quinazolin-6-yl}-allyl)-acetamide;
[3-Methyl-4-(pyridin-3-yloxy)-phenyl]-(6-piperidin-4-ylethynyl-quinazolin-4-yl)-amine;
2-Methoxy-N-(1-{4-[3-methyl-4-(6-methyl-pyridin-3-yloxy)-phenylamino]-quinazolin-6-ylethynyl}-cyclopropyl)-acetamide;
E-N-(3-{4-[3-Chloro-4-(6-methyl-pyridin-3-yloxy)-phenylamino]-quinazolin-6-yl}-allyl)-2-methoxy-acetamide;
N-(3-{4-[3-Chloro-4-(6-methyl-pyridin-3-yloxy)-phenylamino]-quinazolin-6-yl}-prop-2-ynyl)-acetamide;
N-(3-{4-[3-Methyl-4-(6-methyl-pyridin-3-yloxy)-phenylamino]-quinazolin-6-yl}-prop-2-ynyl)-acetamide;
E-N-(3-{4-[3-Chloro-4-(6-methyl-pyridin-3-yloxy)-phenylamino]-quinazolin-6-yl}-allyl)-acetamide;
E-2-Ethoxy-N-(3-{4-[3-methyl-4-(6-methyl-pyridin-3-yloxy)-phenylamino]-quinazolin-6-yl}-allyl)-acetamide;
1-Ethyl-3-(3-{4-[3-methyl-4-(6-methyl-pyridin-3-yloxy)-phenylamino]-quinazolin-6-yl}-prop-2-ynyl)-urea;
Piperazine-1-carboxylic acid (3-{4-[3-methyl-4-(6-methyl-pyridin-3-yloxy)-phenylamino]-quinazolin-6-yl}-prop-2-ynyl)-amide;
(±)-2-Hydroxymethyl-pyrrolidine-1-carboxylic acid (3-{4-[3-methyl-4-(6-methyl-pyridin-3-yloxy)-phenylamino]-quinazolin-6-yl}-prop-2-ynyl)-amide;
2-Dimethylamino-N-(3-{4-[3-methyl-4-(pyridin-3-yloxy)-phenylemino]-quinazolin-6-yl}-prop-2-ynyl)-acetamide;
E-N-(3-{4-[3-Methyl-4-(6-methyl-pyridin-3-yloxy)-phenylamino]-quinazolin-6-yl}-allyl)-methanesulfonamide;
Isoxazole-5-carboxylic acid (3-{4-[3-methyl-4-(6-methyl-pyridin-3-yloxy)-phenylamino]-quinazolin-6-yl}-prop-2-ynyl)-amide;
1-(1,1-Dimethyl-3-{4-[3-methyl-4-(6-methyl-pyridin-3-yloxy)-phenylamino]-quinazolin-6-yl}-prop-2-ynyl)-3-ethyl-urea;
and the pharmaceutically acceptable salts, and solvates of the foregoing compounds.

In one preferred embodiment of the present invention, the compound of formula 2 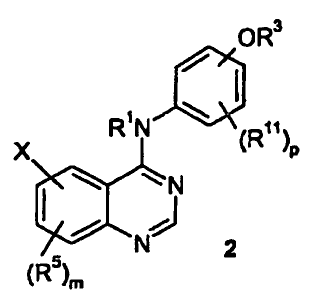 is prepared by reacting a compound of formula 2A 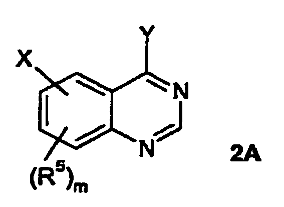 wherein Y is a halide and X, R⁵ and m are as defined for formula 1, with a compound of formula E 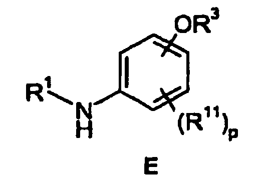 wherein R¹, R³, R¹¹, and p are as defined for formula 1.

In one preferred embodiment the process of present invention is used to make compounds of formula 1, wherein X is Br or I, R⁴ is -C=C-(CR¹⁶R¹⁷)ₜ-R⁸, or -C=C-(CR¹⁶R¹⁷)ₖR¹³ and said reaction is carried out in the presence of a palladium or nickel catalyst selected from the group consisting of Pd(OAc)₂, Pd₂(dba)₃, PdCl₂, Pd(MeCN)₂Cl₂, Pd(PhCN)₂Cl₂, PdCl₂(PPh₃)₂, Pd(PPh₃)₄, BnPdCl(PPh₃)₂, Pd(Otfa)₂, Pd(PPh₃)₂(Otfa)₂, PdCl₂(dppf), Pd(acac)₂, Pd₂(dba)₃-CHCl₃, NI(PPh₃)₄, Pd(dppb), 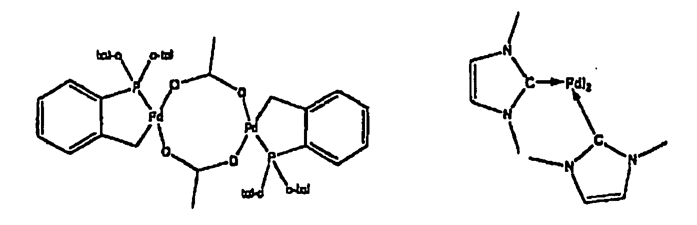 and

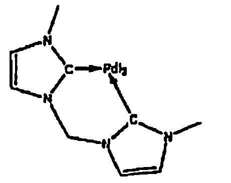

In a preferred embodiment of the process of the present invention the palladium catalyst is selected from the group consisting of Pd(OAc)₂, Pd₂(dba)₃, and Pd(PPh₃)₄.

In a more preferred embodiment of the process of the present invention the palladium catalyst is selected from the group consisting of Pd(OAc)₂ and Pd(PPh₃)₄.

In a preferred embodiment of the process of the present invention the ligand is selected from the group consisting a polymer bound phosphine. 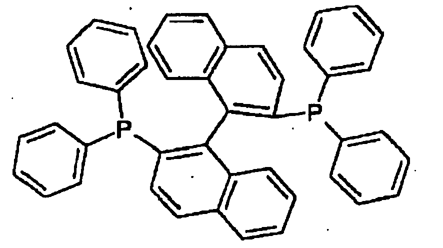 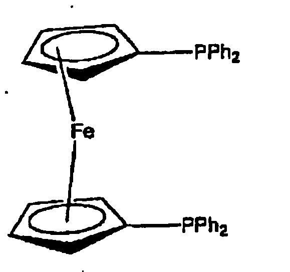 2-methyl-2'-(dicyclohexylphosphino)biphenyl, 2-dimethylamino-2'-(dicyclohexophosphino)biphenyl, and P(R²²)₃, wherein each R²² is independently selected from the group consisting of 2-methyl-2'-(dicyclohexylphosphino)biphenyl, 2-dimethylamino-2'-(dicyclohexylphosphino)biphenyl, phenyl, *o*-toluyl, OMe, and furyl,

In a more preferred embodiment of the process of the present invention the ligand is selected from the group consisting of PPh₃, P(*o*-Tol)₃, P(*o*-OMePh)₃, P(2-Furyl)₃, 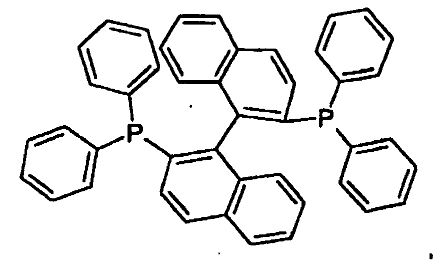 and

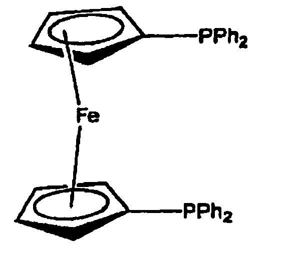

In a most preferred embodiment of the process of the present invention the ligand is selected from the group consisting of PPh₃, P(*o*-Tol)₃, and P(2-Furyl)₃.

In a preferred embodiment of the process of the present invention M is selected from the group consisting of H, Al(R²⁰)₂, Sn(R²¹)₃, MgW, and ZnW and wherein said base is selected from the group consisting of (R)₃N, (R)zNH, RNH₂, OX, Q₂CO₃, Q₃PO₄, QO₂CR, wherein Q is selected from the group consisting of (R)₄N, Na, K, Cs, Cu, Cd, and Ce, and wherein each R is independently selected from H, C₁-C₆ alkyl, -(CR¹R²)ₜ(C₆-C₁₀ aryl), and (CR¹R²)ₜ(4 to 10 membered heterocyclic), wherein t is an integer from 0 to 5, 1 or 2 ring carbon atoms of the heterocyclic group are optionally substituted with an oxo (=0) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₆ alkoxy, and wherein R¹ and R² are as defined for formula 1.

In a preferrerd embodiment of the process of the present invention the base is selected from the group consisting of R₄NF, R₄NCl, R₄NBr, Et₃N, Me₂NEt, iPr₂NEt, CuBr, Cul, CdCl, CsF, K₂CO₃, Na₃PO₄, Na₂HPO₄, NaOAc, DABCO, and 1,6-(dimethylamino)naphthalene, wherein each R is independently selected from H, C₁-C₆ alkyl, -(CR¹R²)₁(C₈-C₁₀ aryl), and -(CR¹R²)₁(4 to 10 membered heterocyclic), wherein t is an integer from 0 to 5, 1 or 2 ring carbon atoms of the heterocyclic group are optionally substituted with an oxo (=0) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₆ alkoxy, and wherein R¹ and R² are as defined for formula 1.

In a preferred embodiment of the process of the present invention the base is selected from the group consisting of NaOEt, NaOMe, NaOH, KOH, LIOH, Ca(OH)₂, TIOH, Ba(OH)₂, Et₃N, Me₂NEt, IPr₂NEt, CuBr, Cul, CdCl, CsF, KF, KCl, K₂CO₃, Na₃PO₄, Na₂HPO₄, NaOAc, DABCO, 1,8-(dimethylamino)naphthalene, R₄NF, R₄NCl, and R₄NBr wherein each R is independently selected from H, C₁-C₆ alkyl, -(CR¹R²)ₜ(C₈-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₈ alkyl, C₂-C₈ alkenyl, C₂-C₈ alkynyl, and C₁-C₈ alkoxy, and wherein R¹ and R² are as defined for formula 1.

In a more preferred embodiment of the process of the present invention the base is selected from the group consisting of Et₃N, Me₂NEt, IPr₂NEt, CuBr, Cul, CdCl, CsF, R₄NF, R₄NCl, R₄NBr, K₂CO₃, Na₃PO₄, Na₂HPO₄, NaOAc, DABCO, and 1,8-(dimethylamino)naphthalene, wherein each R is independently selected from H, C₁-C₈ alkyl, -(CR¹R²)ₜ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₆ alkoxy, and wherein R¹ and R² are as defined for formula 1.

In an even more preferred embodiment of the process of the present invention the base is selected from the group consisting of Et₃N, Me₂NEI, K₂CO₃, Na₃PO₄ and NaOAc.

In a preferred embodiment of the process of the present invention the reaction is carried out in a solvent selected from the group consisting of toluene, benzene, xylene, dimethylformamide, dimethylacetamide, dioxane, tetrahydrofuran, acetonitrile, N-methylpyrrolidinone, dimethylsulfoxide, dimethoxyethane, CH₂Cl₂, CHCl₃, ClCH₂CH₂Cl, N(C₁-C₆ alkyl)₃, N(benzyl)₃, and mixtures thereof.

In a more preferred embodiment of the process of the present invention the solvent selected from the group consisting of toluene, dimethylformamide, dimethylacetamide, dioxane, tetrahydrofuran, acetonitrile, N-methylpyrrolidinone, dimethoxyethane, ClCH₂CH₂Cl, N(C₁-C₈ alkyl)₃, N(benzyl)₃ and mixtures thereof.

In an even more preferred embodiment of the process of the present invention the solvent is selected from tetrahydrofuran, dioxane, dimethoxyethane, dimethylformamide, dimethylacetamide, and mixtures thereof.

In a most preferred embodiment of the process of the present invention the solvent is tetrahydrofuran, water, or a mixture of tetrahydrofuran and water.

In a preferred embodiment of the process of the present invention the reaction is carried out at a temperature ranging from about 25°C to about 175°C.

In a preferred embodiment of the process of the present invention M is B(R¹⁰)₂ and wherein said base is selected from the group consisting of (R)₃N, (R)₂NH, RNH₂, DABCO, 1,8-(dimethylamino)naphthalene, QX, Q₂CO₃, Q₃PO₄, Q₂HPO₄, QO₂CR, QOH, and QOR, wherein Q is selected from the group consisting of (R)₄N, Na. K, Cs, Cu, Cd, and Ca, and wherein each R is independently selected from H, C₁-C₆ alkyl, -(CR¹R²)ₜ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₈ alkynyl, and C₁-C₆ alkoxy, and wherein R¹ and R² are as defined for formula 1.

In another specific embodiment of the process of the present invention the compound of formula 1 is prepared by reacting a compound of formula 2 with a compound of formula H-C≡C-(CR¹⁸R⁷)ₖR¹³ wherein -(CR¹⁸R¹⁷)ₖR¹³ is selected from the group consisting of -CH₂NHC(O)R¹⁵, -CH₂NHSO₂R¹⁵, and -CH₂NHCO₂R¹⁵ wherein said HC≡CCH₂NHC(O)R¹⁵, HC≡CCH₂NHSO₂R¹⁵, and HC≡CCH₂NHCO₂R¹⁵ are prepared by reacting HC≡CCH₂NH₂ with a compound of formula ClC(O)R¹⁵, ClSO₂R¹⁵, or ClCO₂R¹⁵, respectively.

In another specific embodiment or the process of the present invention the compound of formula 1 is prepared by reacting a compound of formula 2 with a compound of formula M-C=C-(CR¹⁶R¹⁷)ₖR¹³ wherein -(CR¹⁶R¹⁷)ₖR¹³ is selected from the group consisting of -CH₂NHC(O)R¹⁵, -CH₂NHSO₂R¹⁵, and -CH₂NHCO₂R¹⁵ wherein sold H₂C=CCH₂NHC(O)R¹³, H₂C=CCH₂NHSO₂R¹⁵ and H₂C=CCH₂NHCO₂R¹⁵ are prepared by reacting HC=CCH₂NH₂ with a compound of formula ClC(O)R¹³, ClSO₂R¹⁵, or ClCO₂R¹⁵, respectively.

In one embodiment of the process of the present invention the compound of formula 1 is prepared, wherein X is Cl, wherein R⁴ is -C=C-(CR¹⁶R¹⁷)ᵢ-R⁹, or -C=C-(CR¹⁶R¹⁷)ₖR¹³, wherein M is B(R¹⁸)₂, and said reaction is carried out in the presence of a catalyst, ligand, base, and solvent, wherein catalyst, ligand, base, and solvent is selected from one of the following groups:
(i) said catalyst is Pd₂(dba)₃ or Pd(OAc)₂, said ligand is 2-methyl-2'-(dicyclohexylphosphino)biphenyl,
   2-dimethylamino-2'-(dicyclohexylphosphino)biphenyl, and P(R²²)₃, wherein R²² is selected from the group consisting of C₁-C₈ alkyl, 2-methyl-2'-(dicyclohexylphosphino)biphenyl and
   2-dimethylamino-2'-(dicyclohexylphosphino)biphenyl, said base is selected from the group consisting of (R)₄N, M₂CO₃, M₃PO₄, and MX, wherein each R is independently selected from H, C₁-C₈ alkyl, -(CR¹R²)ₜ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₈ alkyl, C₂-C₆ alkenyl, C₂-C₆ alkynyl, and C₁-C₆ alkoxy, and wherein R¹ and R² are as defined for formula 1, wherein M is selected from the group consisting of Na, K, Cs and X is halide, and said solvent is selected from the group consisting of toluene, benzene, xylene, DME, acetone, dioxane, DMF, DMAC, NMP, and ACN; or
(ii) said catalyst is selected from the group consisting of Pd(OAc)₂, PdCl₂, Pd(MeCN)₂Cl₂, Pd(PhCN)₂Cl₂, and PdCl₂(PPh₃)₂, said ligand is Ph₄PX, wherein X is selected from the group consisting of Cl, Br, and I, said base is NaOAc or NN dimethylglycine, and said solvent is selected from the group consisting of DMF, DMAC, water, dioxane, THF, ACN, and NMP; or
(iii) said catalyst is selected from the group consisting of Pd(OAc)₂, PdCl₂, Pd(MeCN)₂Cl₂, and Pd(PhCN)₂Cl₂, said ligand is P(OR)₃, wherein R is selected from the group consisting of Et, iPr, Ph, 2,4-dit-BuPh, and Ar, said base is selected from the group consisting of (R)₄N, M₂CO₃ and MO₂CR, wherein M is selected from the group consisting of Na, K, and Cs, wherein each R is independently selected from H, C₁-C₆ alkyl, -(CR¹R²)ₜ(C₆C₁₀ aryl), and -(CR¹R²)ₜ(4 to 10 membered heterocyclic), wherein t is an integer from 0 to 5. 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₆ alkoxy, and wherein R¹ and R² are as defined for formula 1, and said solvent is selected from the group consisting of DMF, DMAC, water, dioxane, THF, ACN, and NMP; or
(iv) said catalyst is selected from the group consisting of 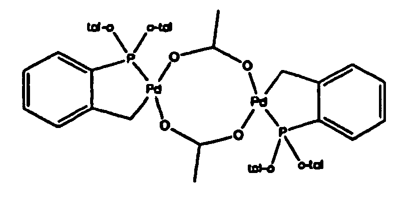 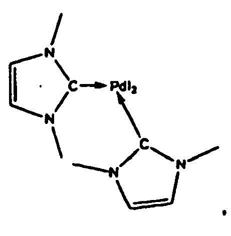 and 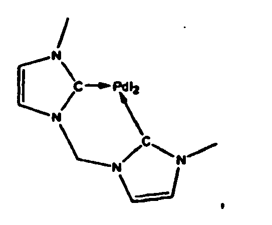 wherein said ligand is P(R²²)₃, wherein R²² is selected from the group consisting of C₁-C₆ alkyl, 2-methyl-2'-(dicyclohexylphosphino)biphenyl and 2-dimethylamino-2'-(dicyclohexylphosphino)biphenyl, said base is (R)₄N or M₂CO₃, wherein M is selected from the group consisting of Na, K, and Cs, wherein each R is independently selected from H, C₁-C₆ alkyl, -(CR¹R²)ₜ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₆ alkoxy, and wherein R¹ and R² are as defined for formula 1 and said solvent is selected from the group consisting of toluene, benzene, xylene, DME, acetone, Dioxane, DMF, DMAC, and NMP; and
(v) said catalyst is Pd₂(dba)₃, said ligand is Ligand 4 or 5, said base is (R)₄N or M₂CO₃, wherein M is selected from the group consisting of Na, K, and Cs, wherein each R is independently selected from H, C₁-C₆ alkyl, -(CR¹R²)ₜ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl. C₂-C₆ alkenyl, C₂-C₆ alkynyl, and C₁-C₈ alkoxy, and wherein R¹ and R² are as defined for formula 1, and said solvent is selected from the group consisting of toluene, benzene, xylene, DME, acetone, Dioxane, DMF, DMAC, and NMP.

In one embodiment of the process of the present invention the compound of formula 1 is prepared, wherein X is chlorine, R⁴ is -C=C-(CR¹⁸R¹⁷)ₜ-R⁸, or -C=C-(CR¹⁸)ₖR¹³ and M is H and said reaction is carried out in the presence of a catalyst, ligand, base, and solvent mixture comprised of one of the following:
(i) said catalyst is Pd₂(dbe)₃ or Pd(OAc)_{2,} said ligand is 2-methyl-2'-(dicyclohexylphosphino)biphenyl, 2-dimethylamino-2'-(dicyclohexylphosphino)biphenyl, and P(R²²)₃, wherein R²² is selected from the group consisting of C₁-C₆ alkyl, 2-methyl-2'-(dicyclohexylphosphino)biphenyl and 2-dimethylamino-2'-(dicyclohexylphosphino)biphenyl, said base is selected from the group consisting of M₂CO₃, M₃PO₄, and MX wherein M is selected from the group consisting of Na, K, Cs, and (R)₄N, wherein each R is independently selected from H, C₁-C₆ alkyl, -(CR¹R²)ₜ(C₈-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₆ alkoxy, and wherein R¹ and R² are as defined for formula 1 and said solvent is selected from the group consisting of toluene, benzene, xylene, DME, acetone, Dioxane, DMF, DMAC, NMP, and ACN; or
(ii) said catalyst is selected from the group consisting of Pd(OAc)₂, PdCl₂, Pd(MeCN)₂Cl₂, Pd(PhCN)₂Cl₂, and PdCl₂(PPh₃)₂, said ligand is Ph₄PX, wherein X is selected from the group consisting of Cl, Br, and I, said base is NaOAc or NN dimethylglycine, and said solvent is selected from the group consisting DMF, DMAC, water, dioxane, THF, ACN, and NMP; or
(iii) said catalyst is selected from the group consisting of 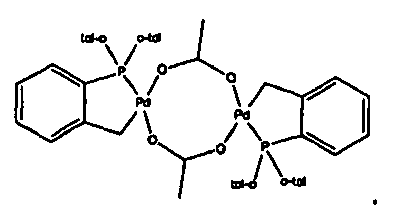 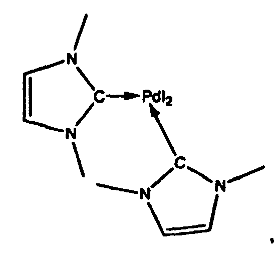 and 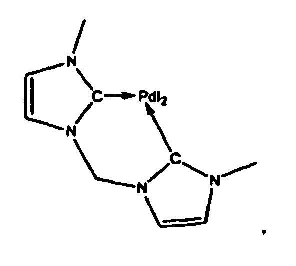 said base is NaOAc, Bu₄NBr, hydrazine, or NaOCHO, and said solvent is selected from the group consisting toluene, benzene, xylene, DME, acetone. Dioxane, DMF, DMAC, and NMP; and
(iv) said catalyst is Pd₂(dba)₃, said ligand is 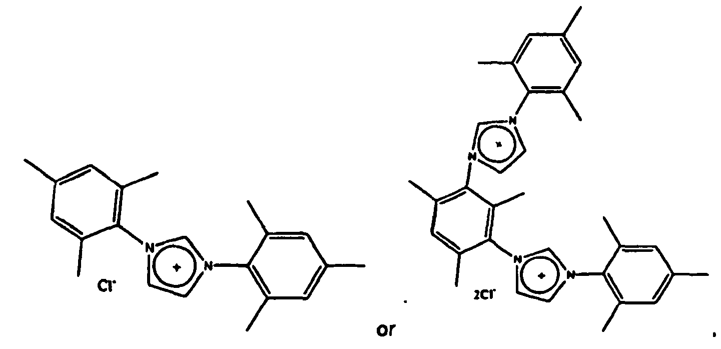 said base is selected from the group consisting NaOAc, Bu₄NBr, hydrazine, and NaOCHO and said solvent is selected from the group consisting toluene, benzene, xylene, DME, acetone, dioxane. DMF, DMAC, and NMP.

in one embodiment of the process of the present invention the compound of formula 1 is prepared, wherein X is chlorine, R⁴ is -C=C-(CR¹⁶R¹⁷)ₜ-R⁸, or -C=C-(CR¹⁶R¹⁷)ₖR¹³ and M is Sn(R)₃, said reaction is carried out in the presence of a catalyst, ligand, base, and solvent mixture, wherein said catalyst is Pd₂(dba)₃ or Pd(OAc)₂, said ligand is P(R²²)₃, wherein R²² is selected from the group consisting of C₁-C₆ alkyl, 2-methyl-2'-(dicyclohexylphosphino)biphenyl and 2-dimethylamino-2'-(dicyclohexylphosphino)biphenyl, said base is selected from the group consisting of M₂CO₃, M₃PO₄, MOH and MX, wherein M is selected from the group consisting of Na, K, Cs, and (R)₄N, wherein each R is independently selected from H, C₁-C₆ alkyl, -(CR¹R²)ₜ(C₈-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₈ alkenyl, C₂₋C₆ alkynyl, and C₁-C₆ alkoxy, and wherein R¹ and R² are as defined for formula 1 and said solvent is selected from the group consisting of DME DMF, DMAC, water, dioxane, THF, ACN, and NMP.

In one embodiment of the process of the present invention the compound fo formula 1 is prepared, wherein X is Br or I, R⁴ is -C≡C-(CR¹⁶R¹⁷)ₜR⁸ or -C≡C-(CR¹⁶R¹⁷)ₜR¹³, said reaction is carried out in the presence of a catalyst, ligand, base, and solvent mixture, wherein said catalyst is selected from the group consisting of Pd(OAc)₂, Pd₂(dba)₃. PdCl₂, Pd(MeCN)₂Cl₂, Pd(PhCN)₂Cl₂, PdCl₂(PPh₃)₂, Pd(PPh₃)₄, Pd(Otfa)₂, Pd(PPh₃)₂(Otfa)₂, PdCl₂(dppf), Pd(acac)₂, Pd₂(dba)₃-CHCl₃, and Pd(dppb), said ligand is selected from the group consisting of PPh₃, P(o-Tol)₃, P(o-OMePh)₃, P(2-Furyl)₃, said base is selected from the group consisting of (R)₂NH, RNH₂, and (R)₃N, wherein each R is independently selected from H, C₁-C₆ alkyl, -(CR¹R²)ₗ(C₆-C₁₀ aryl), and -(CR¹R²)ₜ(4 to 10 membered heterocyclic), wherein t is an integer from 0 to 5, 1 or 2 ring carbon atoms of the heterocyclic group are optionally substituted with an oxo (=O) moiety, the alkyl, aryl and heterocyclic moieties of the foregoing R groups are.optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, and C₁-C₈ alkoxy, and wherein R¹ and R² are as defined for formula 1 and said solvent is selected from the group consisting of toluene, benzene, xylene, dimethylformamide, dimethylacetamide, dioxane, tetrahydrofuran, acetonitrile, N-methylpyrrolidinone, dimethoxyethane, acetone, CH₂Cl₂, CHCl₃, and ClCH₂CH₂Cl.

In one preferred embodiment the Suzuki reaction is employed to prepare the compounds of formula 1 by reacting a compound of formula 2 with a compound of formula M-C=C-(CR¹⁸R¹⁷)ᵣ-R⁹ or M-C=C-(CR¹⁸R¹⁷)ₖR¹³, wherein M ts B. The following review articles, hereby incorporated by reference, identify Pd catalysis and reagents that may be employed in the Suzuki reaction to prepared the compounds of the present invention: (a) Suzuki, A. in Metal-catalyzed Cross-coupling Reactions, Deiderich, F., Stang, P.J., Eds. Wiley, New York, 1998, Chapter 2; and (b) Miyamura, N.; Suzuki, A. Chem. Rev. 1995, 95, 2457-2483. The following articles are hereby incorporated by reference for Suzuki reactions using aryl chlorides: (a) Litike, A.F., Fu, G.C., Angew. Chem. Int. Ed. Engl. 1998, 37, 3387-3388, (b) Wolfe, J.P., Buchwald, S.L., Angew. Chem. Int. Ed. Engl. 1999, 38, 2413-2416, and (c) Litike, A.F., Dai, C., Fu, G.C., J. Am. Chem. Soc., 2000, 122, 4020-4028.

The following table lists preferred combinations of Pd catalysis, ligands, bases and solvents used to prepare the compounds of the present invention using the Suzuki reaction.

| Pd source | Ligand | Base | Solvent |
|---|---|---|---|
| Pd(PPh₃)₄ PdCl₂(PPh₃)₂ Pd(OAc)₂ Pd (Pd/C, Pd black) PdCl₂(dppf) | PAr₃, preferably PPh₃, P(o-Tol)₃, or P(o-OMePh)₃. P(R)(Ar)₂ P(R)₂(Ar) P(R)₃ Dppf, dppe, dppb, or dppp. Polymer bound phosphines | Q(OR)ₙ, wherein n is 1-3, preferably NaOEt. Q(OH)ₙ, preferably NaOH, KOH, LiOH, CaOH₂, TIOH, Ba(OH)₂ (R)₃N preferably Et₃N or Me₂NEt. QF or QCl O(CO₃) OH(PO₄) Q(OCOR), for example NaOAc. | Toluene, benzene, or xylene, DMF, DMAC, water, dioxane, THF, ACN, NMP, MeOH, EtOH, iPrOH, DME, or acetone. |

The following boranes may be employed to provide the vinyl borane coupling partner in the Suzuki reaction from the corresponding acetylene: catecholborane, 9-borabicyclo[3.3.1]nonane [9-BBN], dithexylborane, diisoamylborane, dicyclohexylborane, or HB(OR)₂ wherein R is C₁-C₁₀ alkyl, phenyl, and benzyl or H.

In one preferred embodiment when X is Cl the following Table lists the Pd catalyst, ligand, based and solvent which may be employed for the preparation of the compounds of formula 1. Applicants' also incorporated by reference the related information disclosed in Buchwald, S.L.; Fox, J.M. The Strem Chemiker, Vol 18, no. 1. p. 1-14,2000.

| Pd source | Ligand | Base | Solvent |
|---|---|---|---|
| Pd₂(dba)₃ or Pd(OAc)₂ | P(R)₃, preferably P(*t*-Bu)₃ or P(*i*-Pr)₃. | Q(CO₃) preferably Cs₂(CO₃). | Toluene, benzene, xylene, DME, acetone Dioxane, DMF, DMAC, NMP, or ACN. |
| Pd₂(dba)₃ or Pd(OAc)₂ | 2-methyl-2'-(dicyclo-hexylphosphino)biphe nyl or 2-dimethylamino-2'-(dicyclohexyl-phosphino)biphenyl | O(CO₃), preferably Cs₂(CO₃), QF, preferably CsF, and QPO₄, preferably K₃PO₄. | Toluene, benzene, xylene, DME, acetone Dioxane, DMF, DMAC, NMP, or CAN. |
| Pd(Oac)₂, PdCl₂, Pd(MeCN)₂Cl₂, Pd(PhCN)₂Cl₂, or PdCl₂(PPh₃)₂. | Ph₄PX, wherein X is Cl, Br, or I. | NaOAc or N,N-dimethylglycine | DMF, DMAC, water, dioxane, THF, ACN, or NMP. |
| Pd(OAc)₂, PdCl₂, Pd(MeCN)₂Cl₂, or Pd(PhCN)₂Cl₂. | P(OR)₃, wherein R is Et, iPr, Ph, 2,4-dit-BuPh, Ar, or Dippb. | Q(OCOR), perferably NaOAc, and Q(CO₃), preferably Na(CO₃). | DMF, DMAC, water, dioxane, THF, ACN, or NMP. |
| Palladacycle 1 or Catalysts 1 or 2. | No Ligand | QCO₃, preferably Cs₂CO₃ and K₂CO₃. | Toluene, benzene, xylene. DME, acetone Dioxane. OMF, DMAC, or NMP. |
| Pd₂(dba)₃ | Ligand 4 or 5 | No Base | Toluene, benzene, xylene. DME, acetone Dioxane, DMF, DMAC, or NMP. |

in one preferred embodiment the Heck reaction is employed to prepare the compounds of formula 1 by reacting a compound of formula 2 with a compound of formula M-C=C-(CR¹⁶R¹⁷)ₜ-R⁹ or M-C=C-(CR¹⁶R¹⁷)ₖR¹³ wherein M is H is employed to prepare the compound of formula 1. The following review articles, hereby incorporated by reference, identify reagents that may be employed in the Heck reaction to prepare the compounds of the present invention: (a) Heck, R.F. in Comprehensive Organic Synthesis; Trost, B.M., Ed.; Pergamon: New York, 1991; Vol. 4, Chapter 4.3; (b) Bräse, S.; deMeijere, A. in Metal-catalyzed Cross-coupling Reactions; Deiderich, F.; Stang, P.J., Eds.; Wiley: New York, 1998, Chapter 3; (c) Cabri, W.; Candiani, I. Acc. Chem. Res. 1995, 28, 2-7; and (d) deMeijere, A.; Meyer, F.E. Angew. Chem. Int. Ed. Engl. 1994, 33,2379-2411.

In one preferred embodiment of the process of the present invention the Heck reactions employ aryl chlorides. The following articles disclose the use of aryl chlorides in the Heck reaction, which are hereby incorporated by reference: (a) Riermeier, T.H.; Zapf, A.; Beller, M. Top. Catal. 1997, 4, 301-309; (b) Littke, A.F.; Fu, G.C. J. Org. Chem. 1999, 64, 10-11; (c) Reetz, M.T.; Lohmer, G.; Schwickardi, R. Angew. Chem. Int. Ed. 1998, 37, 481-483; (d) Beller, M,; Zapf, A. Synlett 1998, 792-793; (e) Ben-David, Y.; Portnoy, M.; Gozin, M.; Milstein, D. Organometallics 1992, 11, 1995-1996; (f) Portnoy, M.; Ben-David, Y.; Milstein, D. Organometallics 1993, 12,4734-4735: (g) Portnoy, M.; Ben-David, Y.; Rousso, I.; Milstein, D. Organometallics 1994, 13, 3465-3479; (h) Herrmann, W.A.; Brossmer, C.; Öfele, K.; Reisinger, C.-P.; Priermeler, T.; Beller, M.; Fischer H. Angew. Chem. lnt Ed. Engl, 1995, 34, 1844-1848; (i) Herrmann, W.A.: Elison, M.; Fischer J.; Köcher, C.; Artus, G.R.J. Angew. Chem. Int. Ed. Engl. 1995, 34, 2371-2374; and (j) Herrmann, W.A.; Brossmer, C., Reisinger, C.-P.; Riermeler, T.H.; Ofele, K.; Beller, M. Chem. Eur. J. 1997, 3, 1357-1364.

The following table lists preferred Pd catalysts, ligands, bases, and solvents from Bräse, S.; deMeijere, A. In Metal-catalyzed Cross-coupling Reactions: Deiderich, F.: Stang, P.J., Eds.; Wiley: New York, 1998; Chapter 3, pages 108-109 for use In the Heck reaction.

| Pd source | Ligand | Base | Solvent |
|---|---|---|---|
| Pd(PPh₃)₄ PdCl₂(PPh₃)₂, or BnPdCl(PPh₃)₂. Pd(OAc)₂, Pd(O₂CCF₃)₂, or Pd(PPh₃)₂(O₂CCF₃)₂. Pd (Pd/C, Pd black, Pd on other solid supports such as silica, graphite, clay). PdCl₂, Pd(MeON)₂Cl₂, or Pd(PhCN)₂Cl₂. PdCl₂(dppf), Pd(acac)₂, Pd₂(dba)₃, Pd(dppb), Pd₂(dba)₃-or CHCl₃. P(Ar)₃, preferably PPh₃, P(o-Tol)₃, P(o-OMePh)₃, P(2-Furyl)₃, | PAr₃, preferably PPh₃. P(o-Tol)₃, P(o-OMePh)₃, P(2-Furyl)₃. BINAP, dppf, dppe, dppb, or dppp. Polymer bound phosphines | DABCO, proton sponge, (R)₂NH, (R)NH₂ (R)₃N, QX, wherein X is F. Cl, or Br, Q(CO₃) QH(PO₄) Q(OCOR), preferably NaOAc. | Toluene, benzene. xylene, DMF, DMAC, water, dioxane, THF, ACN, NMP, DMSO, MeOH, EtOH. iPrOH, DME, acetone. CH₂Cl₂, CHCl₃, ClCH₂CH₂Cl, NR₃, preferably NEt₃ or iPr₂NEt. |

In one preferred embodiment when X is Cl the following Table lists the Pd catalyst, ligand, based and solvent, which may be employed for the preparation of the compounds of formula 1 using the Heck reaction.

| Pd source | Ligand | Base | Solvent |
|---|---|---|---|
| Pd₂(dba)₃ or Pd(OAc)₂ | P(R)₃, preferably P(*t*-Bu)₃ or P(*i*-Pr)₃. | Q(CO₃), preferably Cs₂(CO₃). | Toluene, benzene, xylene, DME, acetone Dioxane, DMF, DMAC, NMP, or ACN. |
| Pd(OAc)₂, PdCl₂, Pd(MeCN)₂Cl₂, Pd(PhCN)₂Cl₂, or PdCl₂(PPh₃)₂ | Ph₄PX, wherein X is Cl, Br, or I. | NaOAc or NN dimethylglycine | DMF, DMAC, water, dioxane, THF, ACN, or NMP. |
| Pd(OAc)₂, PdCl₂, Pd(MeCN)₂Cl₂, or Pd(PhCN)₂Cl₂, | P(OR)₃, wherein R is Et, iPr, Ph, 2,4-dit-BuPh, Ar, Or dippb. | Q(OCOR), perferably NaOAc, and Q(CO₃), preferably Na₂CO₃. | DMF, DMAC, water, dioxane, THF, ACN, or NMP. |
| Palladacycle 1 Catalysts 1-2 | No Ligand | NaOAc, Bu₄NBr, hydrazine, or NaOCHO. | DMAc, DMF. or NMP. |
| Pd₂(dba)₃ | Ligand 1 | NaOAc, Bu₄NBr, hydrazine, or NaOCHO. | DMAc, DMF, or NMP. |

In one embodiment of the present invention the Stille Coupling reaction is employed to prepare the compounds of formula 1 by reacting a compound of formula 2 with a compound of formula M-C=C-(CR¹⁶R¹⁷)ₜ-R⁹ or M-C=C-(CR¹⁶R¹⁷)ₖR¹³ wherein M is Sn, in the presence of a ligand, base and solvent. The following review, Mitchell, T.N. in Metal-catalyzed Cross-coupling Reactions; Delderich, F.; Stang, P.J., Eds.; Wiley: New York, 1998, Chapter 4, hereby incorporated by reference, identify reagents that may be employed in the Stille Coupling reaction to prepare the compounds of the present invention.

The following table lists preferred combinations of Pd catalysts, ligands, bases and solvents used to prepare the compounds of the present invention using the Stille Coupling reaction.

| Pd source | Ligand | Base | Solvent |
|---|---|---|---|
| Pd(PPh₃)₄ PdCl₂(PPh₃)₂, or BnPdCl(PPh₃)₂, Pd(Oac)₂, Pd(O₂CCF₃)₂, or Pd(PPh₃)₂(O₂CCF₃)₂. Pd (Pd/C, Pd black, Pd on other solid supports such as silica, graphite, clay). PdCl₂, Pd(MeCN)₂Cl₂, Pd(PhCN)₂Cl₂. PdCl₂(dppf), Pd(acac)₂, Pd₂(dba)₃, Pd(dppb), or Pd₂(dba)₃-CHCl₃. | P(Ar)₃, preferably P(Ph)₃, P(o-Tol)₃, P(o-OMePh)₃, or P(2-Furyl)₃, BINAP, As(Ph)_{3.} Dppf, dppe, dppb, or dppp. | DABCO, proton sponge, (R)₂NH, RNH₂, (R)₃N, preferably Et₃N, or Me₂NEt. QX, wherein X is F, Cl, or Br. CuBr, Cul, or CdCl. Q(CO₃) QH(PO₄) Q(OCOR) perferably NaOAc. | Toluene, benzene, xylene, DMF, DMAC, water, dioxane, THF, ACN, NMP, DMSO, MeOH, EtOH, iPrOH, DME, acetone. CH₂Cl₂, CHCl₃, ClCH₂CH₂Cl, N(R)₃, preferably NEt₃ or iPr₂NEt. |

In one preferred embodiment when X is Cl the following Table lists the Pd catalyst, ligand, based and solvent, which may be employed for the preparation of the compounds of formula 1 using the Stille Coupling reaction.

| Pd source | Ligand | Base | Solvent |
|---|---|---|---|
| Pd₂(dba)₃ or Pd(OAc)₂. | P(R)₃, preferably P(*t*-Bu)₃ or P(*l*-Pr)₃. | QF, preferably CsF, Q(CO₃), preferably Cs₂(CO₃), (R)₃N, Or QOH. | DME DMF, DMAC, water, dioxane, THF, ACN, or NMP. |

In one embodiment of the present invention a metal catalyzed cross coupling reaction is employed to prepare compounds of formula 1 by reacting a compound of formula M-C=C-(CR¹⁶R¹⁷)ₜ-R⁹ or M-C=C-(CR¹⁶R¹⁷)ₖR¹³ wherein M is Mg, Zn, Zr or Al, with a compound of formula 2 in the presence of a ligand, base and solvent. The following table lists preferred combinations of Pd catalysts, ligands, bases and solvents used to prepare the compounds of the present invention using a metal catalyzed cross coupling reaction, wherein M is Mg, Zn, Zr or Al. Applicants also incorporate by reference, Negishi, E-i.; Liu, F. in Metal-catalyzed Cross-coupling Reactions; Deiderich, F.; Stang, P.J., Eds.; Wiley: New York, 1998, Chapter 1.

| Pd source | Ligand | Base | Solvent |
|---|---|---|---|
| Pd(PPh₃)₄ PdCl₂(PPh₃)₂, or BnPdCl(PPh₃)₂. Pd(OAc)₂, Pd(O₂CCF₃)₂, or Pd(PPh₃)₂(O₂CCF₃)₂. Pd (Pd/C, Pd black, Pd on other solid supports such as silica, graphite, clay) PdCl₂, Pd(MeCN)₂Cl₂, or Pd(PhCN)₂Cl₂. PdCl₂(dppf), Pd(acac)₂, Pd₂(dba)₃, Pd(dppb), or Pd₂(dba)₃-CHCl₃. Ni(PPh₃)₄ | PAr₃, preferably P(Ph)₃, P(*o*-Tol)₃, P(o-OMePh)₃, or P(2-Furyl)₃. BINAP. As(Ph)₃ dppf, dppe, dppb, or dppp. Polymer bound phosphines | None, or QX, wherein X is F, Cl, or Br, CuBr, Cul, CdCl, or ZnCl₂. | Toluene, benzene, or xylene. Dioxane or THF. DME CH₂Cl₂, CHCl₃, or ClCH₂CH₂Cl. N(R)₃, preferably NEl₃ or iPrNEt. |

In one embodiment of the present invention employing the Sonogashira Coupling reaction is employed to prepare compounds of formula 1 by reacting a compound of formula H-C-≡C-CR¹⁸R¹⁷)ₜNR⁹ or H-C≡C-(CR¹⁸R¹⁷)ₖR¹³ with a compound of formula 2 in the presence of Cul or CuBr. A ligand, base and solvent are also employed. The following review, Sonogashira, K In Metal-catalyzed Cross-coupling Reactions; Deiderich, F.; Stang, P.J., Eds.; Wiley: New York, 1938; Chapter 5, hereby incorporated by reference, identify reagents that may be employed in the Sonogashira Coupling reaction to prepare the compounds of the present invention. The following table lists preferred combinations of Pd catalysts, ligands, bases and solvents used to prepare the compounds of the present invention using the Sonogashira Coupling reaction.

| Pd source | Ligand | Base | Solvent |
|---|---|---|---|
| Pd(PPh₃)₄ PdCl₂(PPh₃)₂ Pd(OAc)₂, Pd(O₂CCF₃)₂, or Pd(PPh₃)₂(O₂CCF₃)₂. Pd (Pd/C, Pd black, Pd on other solid supports such as silica, graphite, clay) PdCl₂, Pd(MeCN)₂Cl₂, or Pd(PhCN)₂Cl₂. PdCl₂(dppf), Pd(acac)₂, Pd₂(dba)₃, Pd(dppb), or Pd₂(dba)₃-CHCl₃. | P(Ar)₃, preferably P(Ph)_{3,} Polymer bound phosphines | (R)₂NH, preferably, Et₂NH or IPr₂NH. Piperidine, or pyrrolidine. RNH₂, preferably BuNH₂ or IPrNH₂. (R)₃N preferably (EthN or (Me)₂NEt. | Toluene, benzene, or xylene. DMF, DMAC, water, dioxane, THF, ACN, NMP, or DMSO. MeOH or EtOH. DME or acetone. CH₂Cl₂, CHCl₃, or ClCH₂CH₂Cl. N(R)₃, preferably NEt₃ or iPrNEt. |

Preferably, the palladium catalyst employed in the present invention is a palladium (0) catalyst, more preferably the palladium (0) catalyst is tretrakis (triphenylphosphine) palladium (0). This may be added to the reaction mixture directly or generated in situ by adding triphenylphosphine and palladium acetate which is converted to palladium (0) species under the reaction conditions.

This compounds of formula 1 may be used to treat abnormal cell growth in a mammal, including a human, comprising administering to said mammal an amount of a compound of the formula 1, as defined above, or a pharmaceutically acceptable salt, solvate or prodrug thereof, that is effective in treating abnormal cell growth. The abnormal cell growth is cancer, including, but not limited to, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, or a combination of one or more of the foregoing cancers. In other embodiments employing the compounds of formula 1 the abnormal cell growth is a benign proliferative disease, including, but not limited to, psoriasis, benign prostatic hypertrophy or restinosis.

The compounds of formula 1 may be used in the treatment of a disorder associated with angiogenesis in a mammal, including a human, comprising administering to said mammal an amount of a compound of the formula 1, as defined above, or a pharmaceutically acceptable salt, solvate or prodrug thereof, that is effective in treating said disorder. Such disorders include cancerous tumors such as melanoma; ocular disorders such as age-related macular degeneration, presumed ocular histoplasmosis syndrome, and retinal neovascularization from proliferative diabetic retinopathy; rheumatoid arthritis; bone loss disorders such as osteoporosis, Paget's disease, humoral hypercalcemia of malignancy, hypercalcemia from tumors metastatic to bone, and osteoporosis induced by glucocorticoid treatment; coronary restenosis; and certain microbial infections including those associated with microbial pathogens selected from adenovirus, hantaviruses, *Borrelia burgdorferi, Yersinia spp., Bordetella pertussis,* and group A *Streptococcus.*

"Abnormal cell growth", as used herein, unless otherwise indicated, refers to cell growth that is independent of normal regulatory mechanisms (e.g., loss of contact inhibition). This includes the abnormal growth of: (1) tumor cells (tumors) that proliferate by expressing a mutated tyrosine kinase or overexpression of a receptor tyrosine kinase; (2) benign and malignant cells of other proliferative diseases in which aberrant tyrosine kinase activation occurs; (4) any tumors that proliferate by receptor tyrosine kinases; (5) any tumors that proliferate by aberrant serine/threonine kinase activation; and (6) benign and malignant cells of other proliferative diseases in which aberrant serine/threonine kinase activation occurs..

The term "treating", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above.

The term "halo", as used herein, unless otherwise indicated, includes fluoro, chloro, bromo or iodo. Preferred halo groups are fluoro and chloro.

The term "alkyl", as used herein, unless otherwise indicated, includes saturated monovalent hydrocarbon radicals having straight, cyclic (including mono- or multi-cyclic moieties) or branched moieties. It is understood that for said alkyl group to include cyclic moieties it must contain at least three carbon atoms.

The term "cycloalkyl", as used herein, unless otherwise indicated, includes saturated monovalent hydrocarbon radicals having cyclic (including mono- or multi-cyclic) moieties.

The term "alkenyl", as used herein, unless otherwise indicated, includes alkyl groups, as defined above, having at least one carbon-carbon double bond.

The term "alkynyl", as used herein, unless otherwise indicated, includes alkyl groups, as defined above, having at least one carbon-carbon triple bond.

The term "aryl" or "Ar", as used herein, unless otherwise indicated, includes an organic radical derived from an aromatic hydrocarbon by removal of one hydrogen, such as phenyl or naphthyl. "Aryl" or "Ar" are optionally substituted with 1 to 4 substituents independently selected from halo, cyano, nitro, trifluoromethyl, trifluoromethoxy, azido, -OR⁶, -C(O)R⁶, -C(O)OR⁶, -OC(O)R⁶, -NR⁶C(O)R⁷, -C(O)NR⁶R⁷, -NR⁶R⁷, -NR⁶OR⁷, C₁-C₆ alkyl, C₂-C₆ alkenyl, C₂-C₆ alkynyl, -(CR¹R²)ₜ(C₆-C₁₀ aryl), and -(CR¹R²)ₜ(4 to 10 membered heterocyclic), wherein t is an integer from 0 to 5, wherein t, R¹, R², R⁶, and R⁷ are as defined for formula 1.

The term "alkoxy", as used herein, unless otherwise indicated, includes -O-alkyl groups wherein alkyl is as defined above.

The term "4 to 10 membered heterocyclic", as used herein, unless otherwise indicated, includes aromatic and non-aromatic heterocyclic groups containing one or more heteroatoms each selected from O, S and N, wherein each heterocyclic group has from 4 to 10 atoms in its ring system. Non-aromatic heterocyclic groups include groups having only 4 atoms in their ring system, but aromatic heterocyclic groups must have at least 5 atoms in their ring system. The heterocyclic groups include benzo-fused ring systems and ring systems substituted with one or more oxo moieties. An example of a 4 membered heterocyclic group is azetidinyl (derived from azetidine). An example of a 5 membered heterocyclic group is thiazolyl and an example of a 10 membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thlomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the compounds listed above, may be C-attached or N-attached where such is possible. For instance, a group derived from pyrrole may be pyrrol-1-yl (N-attached) or pyrrol-3-yl (C-attached).

The term "Me" means methyl, "Et" means ethyl, and "Ac" means acetyl.

The term" " DME", as used herein, unless otherwise indicated, means dimethoxyethane.

The term "DMF", as used herein, unless otherwise indicated, means dimethylformamide.

The term ''DMAC", as used herein, unless otherwise indicated, means dimethylacetamide.

The term "ACN", as used herein, unless otherwise indicated, means acetonitrile.

The term "NMP", as used herein, unless otherwise indicated, means N-methylpyrrolidinone.

The term "DMSO", as used herein, unless otherwise indicated, means dimethylsulfoxide.

The term "BINAP", as used herein, unless otherwise indicated, is represented by the following formula:

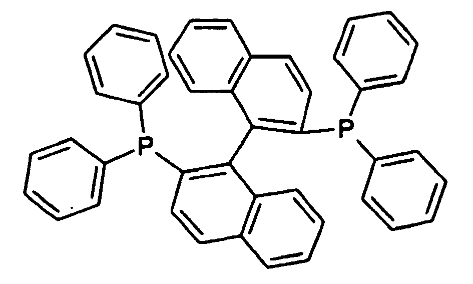

The term "DABCO", as used herein, unless otherwise indicated, means 1,4-diazabicyclo[2.2.2]octane.

The term "DBA", as used herein, unless otherwise indicated, means dibenzanthracene.

The term "dppe", as used herein, unless otherwise indicated, means Ph₂P(CH₂)₂PPh₂.

The term "dppp", as used herein, unless otherwise indicated, means Ph₂P(CH₂)₃PPh₂.

The term "dppb", as used herein, unless otherwise indicated, means Ph₂P(CH₂)₄PPh₂.

The term "dippb ", as used herein, unless otherwise indicated, means *i*Pr₂P(CH₂)₄P*i*Pr₂.

The term "dppf", as used herein, unless otherwise indicated, is represented by the following formula:

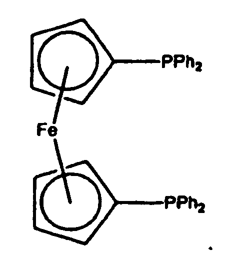

The term "paladacycle 1", as used herein, unless otherwise indicated, is represented by the following formula:

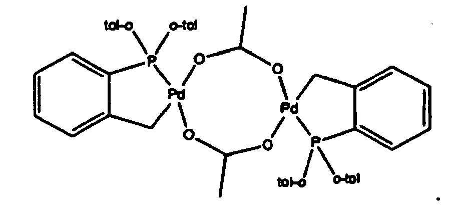

The term "protein sponge", as used herein, unless otherwise indicated, means 1,8-bis(dimethylamino)naphthalene.

The term "catalyst 1", as used herein, unless otherwise indicated, is represented by the following formula:

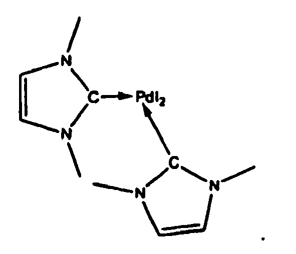

The term " catalyst 2", as used herein, unless otherwise indicated, is represented by the following formula

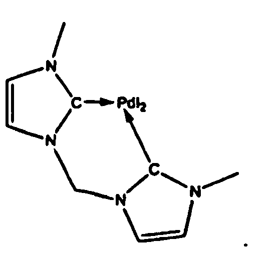

The term "ligand 1", as used herein, unless otherwise indicated, is represented by the following formula

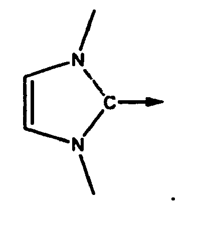

The term "ligand 4", as used herein, unless otherwise indicated, is represented by the following formula

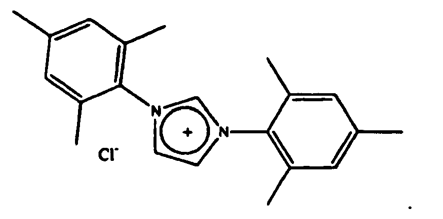

The term "ligand 5", as used herein, unless otherwise indicated, is represented by the following formula

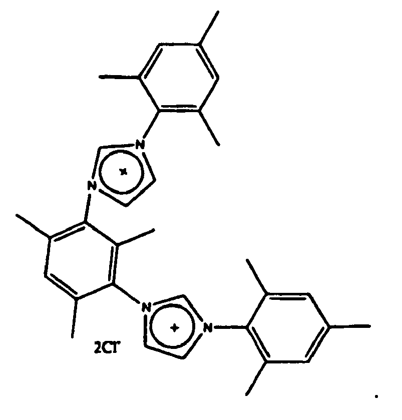

The term" Otfa", as used herein, unless otherwise indicated, means O₂CCF₃.

The term "R", as used herein, unless otherwise indicated, means it is independently selected from H, C₁-C₆ alkyl, -(CR¹R²)ₜ(C₆-C₁₀ aryl); and -(CR¹R²)ₜ(4 to 10 membered heterocyclic), wherein t is an integer from 0 to 5,1 or 2 ring carbon atoms of the heterocyclic group are optionally substituted with an oxo (=O) moiety, the alkyl, aryl and heterocyclic moieties of the foregoing R groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₈ alkynyl, and C₁-C₈ alkoxy, wherein R¹ and R² are as defined above for formula 1.

The phrase "pharmaceutically acceptable salt(s)", as used herein, unless otherwise indicated, includes salts of acidic or basic groups which may be present in the compounds of the present invention. The compounds of formula I that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, such as the hydrochloride, hydrobromide, hydrolodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzonesulfanate, p-toluenesulfonate and pamoate [i.e, 1,1'-methylene-bis-(2-hydroxy-3-naphthoate)] salts. The compounds or formula I that include a basic moiety, such as an amino group, may form pharmaceutically acceptable salts with various amino acids, in addition to the acids mentioned above.

Those compounds of formula I that are acidic in nature are capable of forming base salts with various pharmacologically acceptable cations. Examples of such salts include the alkali metal or alkaline earth metal salts and, particularly, the calcium, magnesium, sodium and potassium salts of the compounds of formula I.

Certain functional groups contained within the compounds of formula I can be substituted for bioisosteric groups, that is, groups which have similar spatial or electronic requirements to the parent group, but exhibit differing or improved physicochemical or other properties. Suitable examples are well known to those of skill in the art, and Include, but are not limited to moieties described in Patini at al., Chem. Rev, 1996, 96, 3147-3176 and references cited therein.

The compounds of formula I have asymmetric centers and therefore exist in different enantiomeric and diastereomeric forms. This invention relates to the use of all optical isomers and stereoisomers of the compounds of formula I and mixtures thereof, and to all pharmaceutical compositions and methods of treatment that may employ or contain them. The compounds of formula 1 may also exist as tautomers. This invention relates to the use of all such lautomers and mixtures thereof.

Isotopically-labelled compounds, and the pharmaceutically acceptable salts, solvates and prodrugs thereof, are identical to those recited in formula 1, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³⁵S, ¹⁶F, and ³⁸Cl, respectively. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavler isotopes such as deuterium, i.e., ²H, can afford certain therapeutic advantages resulting from greater metabolic stability, for example increased i*n vivo* half-life or reduced dosage requirements and, hence, may be preferred in some circumstances. Isotopically labelled compounds of formula 1 and prodrugs thereof can generally be prepared by carrying out the procedures disclosed in the Schemes and/or in the Examples and Preparations below, by substituting a readily available isotopically labelled reagent for a non-isotopically labelled reagent.

Compounds of formula 1 having free amino, amido, hydroxy or carboxylic groups can be converted into prodrugs. Prodrugs include compounds wherein an amino acid residue, or a polypeptide chain of two or more (e.g., two, three or four) amino acid residues is covalently joined through an amide or ester bond to a free amino, hydroxy or carboxylic acid group of compounds of formula 1. The amino acid residues include but are not limited to the 20 naturally occurring amino acids commonly designated by three letter symbols and also includes 4-hidroxyproline, hydroxylysine, demosine, isodemosine, 3-methylhistidine, norvalin, beta-alanine, gamma-aminobutyric acid, citrulline homocysteine, homoserine, omithine and methionine sulfone, Additional types of prodrugs are also encompassed. For instance, free carboxyl groups can be derivatized as amides or alkyl esters. Free hydroxy groups may be derivatized using groups including but not limited to hemisuccinates, phosphate esters, dimethylaminoacetates, and phosphoryloxymethyloxycarbonyls, as outlined in Advanced Drug Delivery Reviews, 1996, 19,115. Carbamate prodrugs of hydroxy and amino groups are also included, as are carbonate prodrugs, sulfonate esters and sulfate esters of hydroxy groups. Derivatization of hydroxy groups as (acyloxy)methyl and (acyloxy)ethyl ethers wherein the acyl group may be an alkyl ester, optionally substituted with groups including but not limited to ether, amine and carboxylic acid functionalities, or where the acyl group is an amino acid ester as described above, are also encompassed. Prodrugs of this type are described in J. Med. Chem. 1996, 39, 10. Free amines can also be derivatized as amides, sulfonamides or phosphonamides. All of these prodrug moieties may incorporate groups including but not limited to ether, amine and carboxylic acid functionalities.

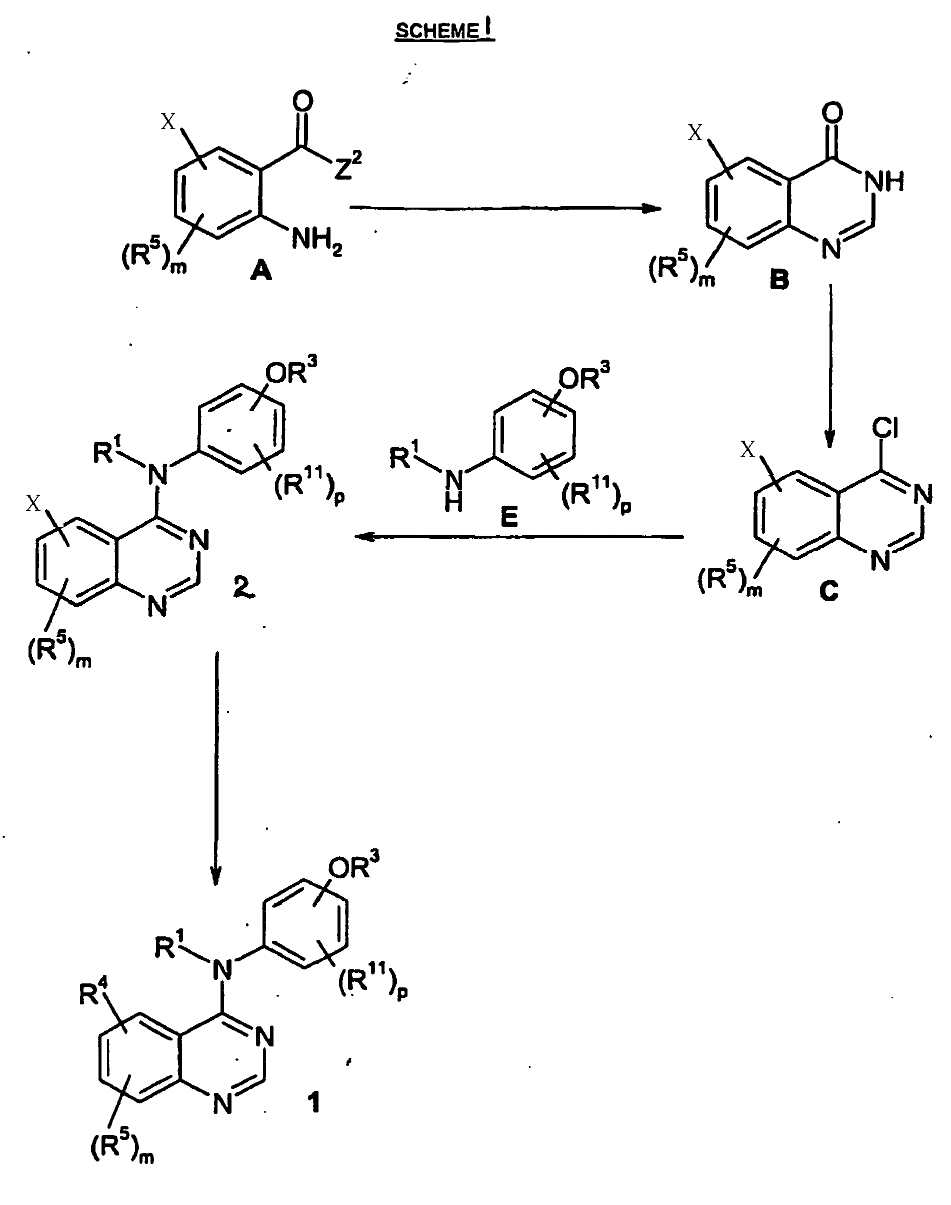

### Detailed Description Of The Invention

General synthetic methods which may be referred to for preparing the compounds of Formula I are provided in United States patent 5,747,498 (issued May 5, 1998), United States patent application serial number 08/953078 (filed October 17, 1997), WO 98/02434 (published January 22, 1998), WO 98/02438 (published January 22, 1998), WO 96/40142 (published December 19, 1996), WO 96/09294 (published March 6, 1996), WO 97/03069 (published January 30, 1997). WO 95/19774 (published July 27, 1995) and WO 97/13771 (published April 17, 1997). Additional procedures are referred to In United States patent application numbers 09/488,350 (filed January 20, 2000) and 09/488,378 (filed January 20, 2000). The foregoing patents and patent applications are incorporated herein by reference in their entirety. Certain starting materials may be prepared according to methods familiar to those skilled in the art and certain synthetic modifications may be done according to methods familiar to those skilled in the art A standard procedure for preparing 6-iodoquinazolinone is provided in Stevenson, T. M., Kazmierczak, F., Leonard, N. J., J. Org. Chem. 1986, 51, 5, p. 616. Pelladium-catalyzed boronic acid couplings are described In Mlyaura, N., Yanagi, T., Suzuki, A. Syn. Comm. 1981, 11, 7, p. 513. Palladium catalyzed Heck couplings are described In Heck et, al. Organic Reactions, 1982, 27, 345 or Cabri et. al. in Acc. Chem. Res. 1995, 28, 2. For examples of the palladium catalyzed coupling of terminal alkynes to aryl halides see: Castro et. al. J. Org. Chem. 1963, 28, 3136. or Sonogashira et. al. Synthesis, 1977, 777. Terminal alkyne synthesis may be performed using appropriately substituted/protected aldehydes as described In: Colvin, E. W. J. et. al. Chem. Soc. Perkin Trans. I, 1977, 869; Gilbert, J. C. et. al. J. Org. Chem., 47, 10, 1982; Hauske, J. R. et. al. Tet. Lett., 33, 26, 1992, 3715; Ohlra, S. et. al. J. Chem. Soc. Chem. Commun., 9, 1992, 721; Trost, B. M. J. Amer. Chem. Soc., 119, 4, 1997, 698; or Marshall, J. A. et. al. J. Org. Chem., 62, 13, 1997, 4313.

Atlernatively terminal alkynes may be prepared by a two step procedure. First, the addition of the lithium anion of TMS (trimethylsilyl) acetylene to an appropriately substituted/protected aldehyde as In: Nakatani, K. et. al. Tetrahedron, 49, 9, 1993, 1901. Subsequent deprotection by base may then be used to Isolate the intermediate terminal alkyne as in Malacria, M.; Tetrahedron, 33, 1977, 2813; or White, J. D. et. at. Tel. Lett., 31, 1. 1990, 59.

Starting materials, the synthesis of which is not specifically described above, are either commercially available or can be prepared using methods well known to those of skill in the art

In each of the reactions discussed or illustrated In the Scheme above, pressure is not critical unless otherwise Indicated. Pressures from about 0.5 atmospheres to about 5 atmospheres are generally acceptable, and ambient pressure, i.e., about 1 atmosphere, Is preferred as a matter of convenience.

With reference to Scheme 1 above, the compound of formula 2 may be prepared by coupling the compound of formula C wherein X and R⁶ are defined above, with an amine of formula E wherein R¹, R³ and R¹¹ are as defined above, in an anhydrous solvent, in particular a solvent selected from DMF (N,N-dimethylformamide), DME (ethylene glycol dimethyl ether), OCE (dichloroethane) and *t*-butanol, and phenol, or a mixture of the foregoing solvents, a temperature within the range of about 50-150°C for a period ranging from 1 hour to 48 hours. The heteroaryloxyanilines of formula E may be prepared by methods known to those skilled In the art, such as, reduction of the corresponding nitro intermediates. Reduction of aromatic nitro groups may be performed by methods outlined In Brown, R. K., Nelson, N. A. J. Org. Chem. 1954, p. 5149; Yuste, R., Saldana, M, Walls, F., Tet. Lett. 1982, 23, 2, p. 147; or In WO 96/09294, referred to above. Appropriate heteroaryloxy nitrobenzene derivatives may be prepared from halo nitrobenzene precursors by nucleophilic displacement of the halide with an appropriate alcohol as described In Dinsmore, C.J. et. al., Bloorg. Med. Chem. Lett., 7, 10, 1997, 1345; Loupy, A. et. al., Synth. Commun., 20, 18, 1990, 2855; or Brunelle, D. J., Tet. Lett., 25, 32, 1984, 3383. Compounds of formula E In which R¹ is a C₁-C₈ alkyl group may be prepared by reductive amination of the parent aniline with R¹CH(O). The compound of formula C can be prepared by treating a compound of formula B with a chlorinating reagent such as POCl₃, SOCl₂ or ClC(O)C(O)Cl/DMF In a halogenated solvent at a temperature ranging from about 60°C to 150°C for a period ranging from about 2 to 24 hours. Compounds of formula B may be prepared from a compound of formula A wherein X is as described above and Z² Is NH₂, C₁-C₈ alkoxy or OH, according to one or more procedures described In WO 95/19774, referred to above.

Any compound of formula 1 can be converted Into another compound of formula 1 by standard manipulations to the R⁴ group. These methods are known to those skilled in the art and Include a) removal of a protecting group by methods outlined In T. W. Greene and P.G.M. Wuts, "Protective Groups In Organic Synthesis", Second Edition, John Wiley and Sons, New York, 1991; b) displacement of a leaving group (halide, mesylale, tosylate, etc) with a primary or secondary amine, thiol or alcohol to form a secondary or tertiary amine, thioether or ether, respectively, c) treatment of phenyl (or substituted phenyl) carbamates with primary or secondary amines to form the corresponding ureas as In Thavonekham, B et al. Synthesis (1997), 10, p1189; d) reduction of propargyl or homopropargyl alcohols or N-BOC protected primary amines to the corresponding E-allylic or E-homoallytic derivatives by treatment with sodium bis(2-methoxyethoxy)aluminum hydride (Red-Al) as In Denmark, S. E.; Jones, T. K. J. Org. Chem. (1982) 47, 4595-4597 or van Benthem, R. A. T. M.; Michels, J. J.; Speckamp, W. N. Synlett (1994), 368-370; e) reduction of alkynes to the corresponding Z-alkene derivatives by treatment hydrogen gas and a Pd catalyst as in Tomassy, B. et. al. Synth. Commun. (1998), 28, p1201 f) treatment of primary and secondary amines with an isocyanate, acid chloride (or other activated carboxylic acid derivative), alkyl/aryl chloroformate or sulfonyl chloride to provide the corresponding urea, amide, carbamate or sulfonamide; g) reductive amination of a primary or secondary amine using R¹CH(O); and h) treatment of alcohols with an isocyanate, acid chloride (or other activated carboxylic acid derivative), alkyl/aryl chloroformate or sulfonyl chloride to provide the corresponding carbamate, ester, carbonate or sulfonic acid ester.

The compounds of formula I may have asymmetric carbon atoms. Diasteromeric mixtures can be separated Into their Individual diastereomers on the basis of their physical chemical differences by methods known to those skilled in the art, for example, by chromatography or fractional crystallization. Enantiomers can be-separated by converting the enantiomeric mixtures Into a diastereomric mixture by reaction with an appropriate optically active compound (e.g., alcohol), separating the diastereomers and converting (e.g., hydrolyzing) the individual diastereomers to the corresponding pure enantiomers. All such isomers, including diastereomeric mixtures and pure enantiomers are considered as part of the invention.

The compounds of formula 1 that are basic in nature are capable of forming a wide variety of different salts with various inorganic and organic acids. Although such salts must be pharmaceutically acceptable for administration to animals, it is often desirable In practice to initially Isolate the compound of formula 1 from the reaction mixture as a pharmaceutically unacceptable salt and then simply convert the latter back to the free base compound by treatment with an alkaline reagent and subsequently convert the letter free base to a pharmaceutically acceptable acid addition salt. The acid addition salts of the base compounds of formula I are readily prepared by treating the base compound with a substantially equivalent amount of the chosen mineral or organic acid in an aqueous solvent medium or In a suitable organic solvent, such as methanol or ethanol. Upon careful evaporation of the solvent, the desired solid salt Is readily obtained. The desired acid salt can also be precipitated from a solution of the free base in an organic solvent by adding to the solution an appropriate mineral or organic add.

Those compounds of formula 1 that are acidic In nature are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of formula 1. Such non-toxic base salts include those derived from such pharmacologically acceptable cations as sodium, potassium calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product. Since a single compound of formula I may include more than one acidic or basic moieties, the compounds of formula I include mono, di or tri-salts in a single compound.

Administration of the compounds of formula I (hereinafter the "active compound(s)") can be effected by any method that enables delivery of the compounds to the site of action. These methods include oral routes, intraduodenal routes, parenteral injection (including intravenous, subcutaneous, intramuscular, intravascular or infusion), topical, and rectal administration.

The amount of the active compound administered will be dependent on the subject being treated, the severity of the disorder or condition, the rate of administration, the disposition of the compound and the discretion of the prescribing physician. However, an effective dosage is in the range of about 0.001 to about 100 mg per kg body weight per day, preferably about 1 to about 35 mg/kg/day, in single or divided doses. For a 70 kg human, this would amount to about 0.05 to about 7 g/day, preferably about 0.2 to about 2.5 g/day. In some instances, dosage levels below the lower limit of the aforesaid range may be more than adequate, while In other cases still larger doses may be employed without causing any harmful side effect, provided that such larger doses are first divided into several small doses for administration throughout the day.

The active compound may be applied as a sale therapy or may involve one or more other anti-tumour substances, for example those selected from, for example, mitotic Inhibitors, for example vinblastine; alkylating agents, for example cis-platin, carboplatin and cyclophosphamide; anti-metabolites, for example 5-fluorouracil, cytosine arabinoside and hydroxyurea, or, for example, one of the preferred anti-metabolites disclosed In European Patent Application No. 239362 such as N-(5-[N-(3,4-dihydro-2-methyl-4-oxoquinazolin-8-ylmethyl)-N-methylamino]-2-thenoyl)-L-glutamic acid; growth factor inhibitors; cell cycle inhibitors; intercalating antibiotics, for example adriamycin and bleomycin; enzymes, for example interferon; and anti-hormones, for example anti-estrogens such as Nolvadex^{™} (tamoxifen) or, for example anti-androgens such as Casodex^{™} (4'-cyano-3-(4-fluorophenylsulphonyl)-2-hydroxy-2-methyl-3'-(trifluoromethyl)propionanilide). Such conjoint treatment may be achieved by way of the simultaneous, sequential or separate dosing of the individual components of the treatment.

The pharmaceutical composition may, for example, be In a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be In unit dosage forms suitable for single administration of precise dosages. The pharmaceutical composition will include a conventional pharmaceutical carrier or excipient and a compound according to the invention as an active ingredient. In addition, It may include other medicinal or pharmaceutical agents, carriers, adjuvants, etc.

Exemplary parenteral administration forms Include solutions or suspensions of active compounds in sterile aqueous solutions, for example, aqueous propylene glycol or dextrose solutions. Such dosage forms can be suitably buffered, if desired.

Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed In soft and hard filled gelatin capsules. Preferred materials, therefor, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled In this art. For examples, see Remington's Pharmaceutical Sciences, Mack Publishing Company, Easter, Pa., 15th Edition (1975).

The examples and preparations provided below further illustrate and exemplify the methods of preparing compounds of formula I. It is to be understood that the scope of the present invention is not limited In any way by the scope of the following examples and preparations. In the following examples molecules with a single chiral center, unless otherwise noted, exist as a racemic mixture. Those molecules with two or more chiral centers, unless otherwise noted, exist as a racemic mixture of diastereomers. Single enantiomers/diastereomers may be obtained by methods known to those skilled in the art.

Where HPLC chromatography is referred to in the preparations and examples below, the general conditions used, unless otherwise indicated, are as follows. The column used is a ZORBAX^{™} RXC18 column (manufactured by Hewlett Packard) of 150 mm distance and 4.6 mm interior diameter. The samples are run on a Hewlett Packard-1100 system. A gradient solvent method is used running 100 percent ammonium acetate / acetic acid buffer (0.2 M) to 100 percent acetonitrile over 10 minutes. The system then proceeds on a wash cycle with 100 percent acetonitrile for 1.5 minutes and then 100 percent buffer solution for 3 minutes. The flow rate over this period is a constant 3 mU minute.

The present invention is illustrated by the following Examples. It will be understood, however, that the invention is not limited by the specific details of the following Examples.

### Example 1

### 6-Iodo-[3-methyl-4-(6-methyl-pyridine-3-yloxy)-phenylamino]-quinazoline

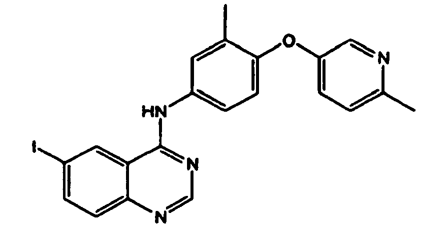

A 3 neck round bottom flask was fitted with a mechanical stirrer and kept under N₂. The flask was charged with the chloroquinazoline (10.0 g, 34.43 mol) and dry THF (35 ml). The 3-amino-4-methylpyridine (7.38 g, 34.43 mmol) and dry THF (45 ml) were added and the yellow suspension was heated to reflux. After 15 min most of the reactants went into solution and a fine yellow suspension was obtained. After 25 min, the internal temperature of the reaction mixture was 56°C, and precipitation of the desired product started. Heating was continued for a further 2 hours and the reaction mixture was allowed to cool to room temperature while remaining in the oil bath. Yellow crystals were collected by filtration, washed with cold (0°C) THF (1 x 10 ml) and dried at 50°C, p < 200 mbar. The title compound was obtained as light yellow crystals (15.75g, 98%). Rf = 0.45 (EtOAC/MeOH = 9/1). ¹H NMR (CDCl₃, 300 MHz): δ = 11.40 (br, s, 1H, NH), 9.29 (d, J = Hz, 1H, H-2), 8.91 (s, 1H, H-2"), 8.36-8.32 (m, 2H, H-7, H-8), 7.74-7.73 (m, 2H, H-4", H-5), 7.62 (dd, J₁ = 8.7Hz, J₂= 2.6Hz, 1H, H-5") 7.49-7.46 (m, 2H, H-6', H-5), 7.06 (d, J = 8.7Hz, 1H, H-2'), 2.54 (s, 3H, CH₃), 2.26 (s, 3H, CH₃), ¹³C NMR (CDCl₃+ D₆-DMSO. 75 MHz): δ = 159.51, 153.63, 153.17, 152.82, 152.70, 145.26, 141.37, 138.01, 134.75, 134.65, 131.05, 129.10, 128.74, 126.77, 124.86, 124.43, 120.41, 116.98, 94.89, 23.54, 17.67.

The title compound had a t_{R} (min) of 12.13 under the following RP-HPLC conditions: Symmetry Shield RP18, 75 x 4.6 mm; Flow 1.0 mL / min; 205/210/220/ 245 nm; Temp. 25°C; Injection Volume: 10 µL of a ca. 0.5% solution in ACN/H₂O 9/1; Eluent: B: ACN, C: 0.01 mmol NH₄OAc in H₂O pH = 6.0; and Gradient: 0 min: B = 30%, C = 70 %; and 20 min: B = 85%, C = 15%.

### Example 2

### 2-Methoxy-acetic acid propargylamide

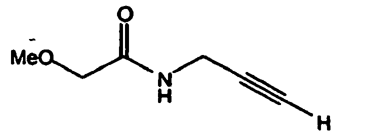

A solution of methoxy acetyl chloride (12.5 ml, 0.137 mol, 1.2 equiv.) in dry CH₂Cl₂ (45 ml) kept under N₂ was cooled to -40°C. A solution of propargylamine (7.98 ml, 0.125 mol, 1.0 equiv.) in dry CH₂Cl₂ (40 ml) was added over 45 minutes keeping the temperature less than-25°C. After 15 minutes triethylamine (17.4 ml, 0.125 mol, 1.0 equiv.) was added over 45 minutes keeping the temperature less than -25°C. The reaction mixture was warmed to room temperature. TLC after 3 hours showed conversion complete. The reaction mixture was quenched with H₂O (50 ml) and the organic phase was washed with half-saturated NaCl solution, filtered through cotton wool and concentrated at a temperature of 40°C and pressure of greater than 650 mbar. The crude compound was purified by short path distillation (boiling point of 49°C and p of 0.09 mbar). The title compound was obtained as a colorless liquid (7.84 g. 50 %) which crystallized upon standing.
R*_{f}* = 0.36 (heptane/EtOAc = 7/3).
¹H NMR (CDCl₃, 300 MHz): δ= 6.72 (br, s, 1H, N-H). 4.09 (dd, J₁=5.5 Hz, J₂= 2.6 Hz, 2H, CH₂-NH), 3.92 (s, 2H, CH₂-OMe), 3.43 (s, 3H, OCH₃), 2.24 (t, J=2.6 Hz, 1H, alkyne CH).
¹³C-NMR (CDCl₃, 75 MHz): δ= 169.14 (C=O). 79.11 (C-2'), 71.63 (C-2). 71.41 (C-3'), 59.04 (OCH₃), 28.26 (C-1').

Gas chromatography was used to determine the t_{R} (min) of 6.42 under the conditions shown in the table below.

| | |
|---|---|
| Column | DB-5 (30 m x 0.32 mm, 0.25 µm film thickness) |
| Injector | Split, initial Temp. 250°C |
| Split ratio | 60.243:1 |
| Split flow | 108.3 ml/min, gas type: hydrogen |
| Oven | 60°C, 1 min, 10°C/min. 290°C. 10 min |
| Inject-Temp | 250°C |
| Detector (FID) | Detector Temp. 250°C |
| Detector flow | H₂: 40,0 ml/min. air: 450 ml/min |
| Makeup flow | N₂: 45.0 ml/min |

### Example 3

### Preparation of 6-(N-Methoxyacetyl-3-amino-propen-1-yl)-4-[3-methyl-4-(6-methyl-pyridine-3-yloxy)-phenylamino]-quinazoline Using Suzuki Coupling Reaction

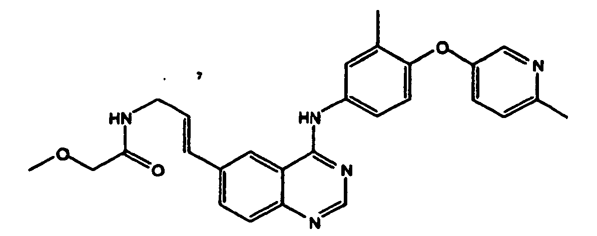

2-methyl-2-butene (0.59 ml, 5.60 mmol, 2.8 equiv.) was added over 1 hour to a cold (0-5°C) solution of BH₃*THF complex (1.0 M sol, 3.0 ml, 3.0 mmol, 1.5 equiv.) kept under N₂. The reaction mixture was stirred at this temperature for 30 minutes followed by the addition of 2-Methoxy-acetic acid propargylamide (255 mg, 2 mmol, 1.0 equiv.) dissolved in dry THF (1 ml) over 15 minutes. The ice-bath was removed and the reaction mixture was warmed to room temperature over 20 minutes. The reaction mixture was then heated at 35°C for 1 hour. K₂CO₃ (0.55 g, 4 mmol, 2.0 equiv.) dissolved in degassed H₂O (1.2 ml) was added over 30 minutes to the reaction mixture. During the addition of the first half gas evolution was observed which seized during further addition. 6-Iodo-[3-methyl-4-(6-methyl-pyridine-3-yloxy)-phenylamino]-quinazoline (1.41 g, 3 mmol, 1.5 equiv.) was added in three portions giving a yellow suspension. PPh₃ (21 mg, 0.08 mmol, 4 mol%) and Pd(OAc)₂ (4.5 mg, 0.02 mmol, 1 mol%) were added each in one portion and the reaction mixture was heated to reflux (65-68°C). After about 30 minutes a yellow solution was obtained and the reaction was monitored by HPLC assay. After 18 hours the reaction mixture was cooled to room temperature followed by the addition of half-saturated NaCl solution (10 ml) and EtOAc (10 ml). The organic phase was separated, washed with H₂O (5 ml) and concentrated at 50°C and a pressure of less than 200 mbar. Purification by plug filtration, SiO₂, EtOAc/MeOH = 9/1. The title compound was obtained as light yellow crystals (0.55 g, 59 %). R_{f} = 0.16 (EtOAc/MeOH = 9/1). ¹H-NMR (CDCl₃, 250 MHz): δ =8.71 (s, 1H, H-2), 8.25 (d, J=1.7 Hz, 1H, H-8), 7.90(s, 1H, H-7), 7.82 (s, 1H, NH), 7.79 (s, 1H, H-5), 7.66 (d, J=2.5Hz. 1H, H-4°), 7.54 (dd, J₁=8.7Hz, J₂=2.6Hz, 1H, H-5°). 7.15-7.07 (m, 2H, H-5'. H-6'), 6.91 (d, J=8.7Hz, 1H, H-2'), 6.83 (bt, 1H, NH), 6.65 (d, J=15.9Hz, 1H. H-9). 6.34 and 6.29 (dt, J₁=15.9Hz, J₂=6.1Hz, 1H, H-10), 4.14 (dt, J=6.1Hz, 2H, CH₂OMe). 3.97 (s, 2H, CH₂NH), 3.45 (s, 3H, OCH₃), 2.53 (s, 3H, CH₃), 2.29 (s, 3H, CH₃). ¹³C-NMR (CDCl₃, 75 MHz): δ = 169.76 (C=O), 157.90, 154.93, 152.367, 152.23, 150.90, 149.74, 139.34, 134.73, 134.63, 131.16, 130.77, 130.36, 128.85, 129.98, 125.47, 124.66, 123.65, 121.32, 119.51, 119.13, 115.39, 71.96, 59.26, 40.84, 23.57, 16.41.

Using reverse phase high performance liquid chromatography t_{R} (min) was found to be 6.02 for the title compound under the conditions shown in the following table.

| | |
|---|---|
| Symmetry Shield RP18 | 75 x 4.6 mm |
| Flow | 1.0 mL / min |
| Wavelength | 205/210/220/245 nm |
| Temp. | 25°C |
| Injection Volume | 10 µL of a ca. 0.5% solution in ACN/H₂O 9/1 |
| Eluent B | ACN |
| Eluent C | 0.01 mmol NH₄OAC in H₂O pH = 6.0 |
| Gradient 0 min | B = 30%. C = 70 % |
| Gradient 20 min | B = 85%. C = 15 % |

### Example 4

### Preparation of 2-Methoxy-acetic acid propargylamide-9-BBN

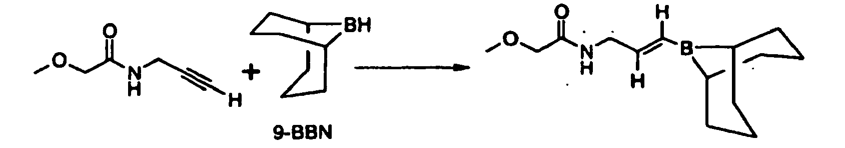

To a solution of 18.88 g of 9-borabicyclononane (9-BBN) in 242 ml of tetrahydrofuran (THF) was added a solution of 19.67 g of 2-methoxy-acetic acid propargylamide and 48.3 ml of THF over 18 minutes at 19°C to 25°C. The reaction mixture was stirred for 21 hours at ambient temperature.

### Example 5

### Preparation of 6-(N-Methoxyacetyl-3-amino-propen-1-yl)-4-[3-methyl-4-(6-methyl-pyridine-3-yloxy)-phenylamino]-quinazoline Using Suzuki Coupling Reaction

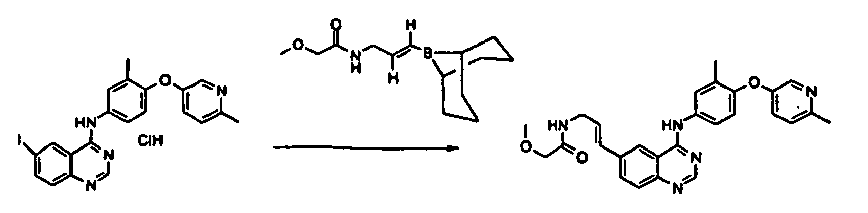

The reaction mixture from Example 4 was cooled to 8.5°C and a solution of 42.77 g of potassium carbonate in 348 ml of water was added over 42 minutes maintaining the pot temperature between 8°C and 10°C. To this reaction was added 24.15 g of (6-iodo-quinazolin-4-yl)-[3-methyl-4-(6-methyl-pyridin-3-yloxy)-phenyl]-amine, 541 mg of triphenylphosphine, and 116 mg of palladium (II) acetate and the mixture was heated to 50°C for 21 hours. The completed reaction was then cooled back to ambient temperature and 290 ml of ethyl acetate (EtOAc) was added and the layers were separated. The product was then extracted from the organic layer into 140 ml of 1 N hydrochloric acid and the product-rich aqueous was washed 4 times with 145 ml of EtOAc. A 1 L round-bottomed flask was charged the product-rich aqueous layer, 290 ml new EtOAc, 2.42 g of KBB Darco (activated carbon, Aldrich, Inc.), 2.42 g filter-aid (Celite, Aldrich, Inc.), and 150 ml of 1N sodium hydroxide. The mixture was stirred for 26 minutes at ambient temperature and then the solids were filtered off through filter-aid and washed with 24 ml of EtOAc. The layers of the filtrate were separated and the product-rich organic layer was washed with 145 ml of water. Next, 140 ml of 30% hydrogen peroxide was charged to the organic layer and stirred for 20 minutes: A solution of 1 g sodium chloride in 4 ml of water was added to the peroxide mixture and the layers were separated. The organic layer was cooled in an ice water bath to 12°C and a solution of 14.5g sodium bisulfite in 131 ml of water was added over 23 minutes maintaining the pot temperature less than 26°C. The layers were separated and to the product-rich acidic sodium bisulfite layer was added 300 ml of EtOAc. The pH of the aqueous layer was adjusted to pH to 10-10.5 with 160 ml of 1N sodium hydroxide and the layers were separated. The product-rich organic layer was washed with 100 ml of water, the layers were separated, and the organic layer was vacuum concentrated to 125 ml volume. The concentrate was displaced under vacuum with 483 ml of EtOAc, and the solids were filtered. The solids were washed with 24 ml of EtOAc, and then vacuum dried at 46°C for 20 hours to provide 10.70 g of light yellow solids. Analytical HPLC assay indicates the solids to be 95.6% 6-(*N*-Methoxyacetyl-3-amino-propen-1-yl)-4-[3-methyl-4-(6-methyl-pyridine-3-yloxy)-phenylamino]-quinazoline by area percent.

### Example 6

### Preparation of 6-(N-Methoxyacetyl-3-amino-propen-1-yl)-4-[3-methyl-4-(6-methyl pyridine-3-yloxy)-phenylamino]-quinazoline Using Heck Coupling Reaction

To a round bottom flask with nitrogen atmosphere, reflux condenser, oil bath, and overhead stirring was charged 5 vol anhydrous DMF and the solvent was purged with nitrogen. To the flask was added 0.04 eq. palladium acetate, the mixture was purged with nitrogen, then 0.08 eq triphenyl phosphine was added and the mixture was again purged with nitrogen. The mixture was stirred to achieve a red solution (-30-60 minutes). After the stirring period, the solution was purged with nitrogen and 2 eq sodium acetate, 1 eq (6-iodo-quinazolin-4-yl)-[3-methyl-4-(6-methyl-pyridin-3-yloxy)-phenyl]-amine and 1.1 eq N-Allyl-2-methoxy-acetamide were charged to the flask. The mixture was again purged with nitrogen and heated to 100°C for 4-6 hours. After reaction completion (as determined by HPLC), the reaction was quenched with 10 vol. 1 N HCl. The aqueous layer was separated and reextracted once with 20 vol. EtOAc. The aqueous layer was basified with 6 N NaOH to pH 9. The product was extracted from the aqueous layer using EtOAC (1x 20 vol. and then 1 x 10 vol.). The organic layer was separated and concentrated to low volume and the remaining ethyl acetate was displaced with acetonitrile. The solution was stirred overnight and the product 6-(*N*-Methoxyacetyl-3-amino-propen-1-yl)-4-[3-methyl-4-(6-methyl-pyridine-3-yloxy)-phenylamino]-quinazoline precipitated out. The solids were then granulated and filtered.

### Example 7

### 6-Chloro-[3-methyl-4-(6-methyl-pyridine-3-yloxy)-phenlyamino]-quinazoline

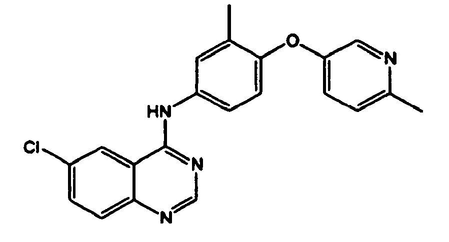

A 3 neck round bottom flask was fitted with a mechanical stirrer and kept under nitrogen. To the flask was charged dry THF (400 ml), dichloroquinazoline (18.6 g, 93.3 mmol) and 3-amino-4-methylpyridine (20.0 g. 93.3 mmol). The light, thin yellow slurry was heated to 50°C and after approximately 4 hours, the reaction was complete by HPLC. The now darker and much thicker yellow slurry was allowed to slowly cool to 20°C. The desired product was collected by filtration and washed with THF (1 x 100 ml). The yellow solids were dried at 20°C with p - 23 inches Hg. The yellow crystals were obtained in 64 % yield (22.41 g). H NMR (CD₃OD, 400 MHz): δ 8.80 (s, 1 H), 8.75 (s, 1H), 8.38 (s, 1H). 8.12-8.10 (dd, 1 H. J=2.0, 2.4), 7.88-7.83 (m, 2 H), 7.76-7.66 (m, 3 H), 7.17 (d, 1 H, J=8.8), 2.68 (s, 3 H), 2.31 (s, 3 H).

The title compound had a t_{R} (min) of 7.213 under the following RP-HPLC conditions: SB-CN 4.6×150 mm; flow = 2 ml/min; λ = 237 nm; temp. = 20°C; injection volumes = 5 µL; eluent = 0.1 % phosphoric acid, 0.3 % triethylamine in DI water / MeCN (60:40); gradient at 5 minutes to 90 % in 5 minutes and then at 10 m

### Example 8

### Preparation of 6-(N-Methoxyacetyl-3-amino-propen-1-yl)-4-[3-methyl-4-(6-methylpyridine-3-yloxy)-phenylamino]-quinazoline Using Heck Coupling Reaction

The Heck reaction was carried out by charging a reaction flask with DMF (5 volumes), tri-t-butylphosphine (0.06 equiv.) and palladium acetate (0.04 equiv.). The reaction was charged with nitrogen and allowed to stir for 30 minutes. After stirring, the reaction was charged with 10.0g (6-chloro-quinazolin-4-yl)-[3-methyl-4-(6-methyl-pyridin-3-yloxy)-phenyl]-amine and 1.1 eq N-Allyl-2-methoxy-acetamide, and sodium acetate (2 equiv.). The reaction was sparged and heated to 100°C for 24 hours. After cooling, the reaction was deemed complete by HPLC. The work-up was followed by charging 1 N HCl (10 volumes) and ethyl acetate (20 volumes). After separation, the aqueous was washed with 1 x 20 vol. ethyl acetate. The aqueous was then charged with 0.05 wt equiv. celite, 0.05 wt. equiv. Darco, 10 vol. ethyl acetate, 0.76 vol. THF and 15 volumes 3 N NaOH. The mixture was stirred for 30 minutes and filtered over celite. The layers were separated and the product layer (organic) was washed 1 x 20 volumes with brine. The product layer was then concentrated to a low volume and any residual water was removed via azeotropic distillation with excess ethyl acetate. The final solution was concentrated to a low volume. The solution was stirred overnight and the product 6-(*N*-Methoxyacetyl-3-amino-propen-1-yl)-4-[3-methyl-4-(6-methylpyridine-3-yloxy)-phenylamino]-quinazoline precipitated out. The solids were then granulated and filtered.

## Claims

1. A method for preparing a compound of formula 1 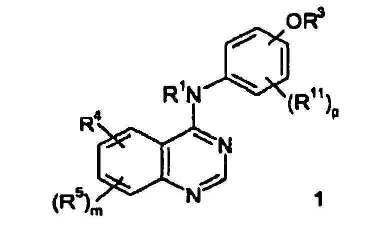 pharmaceutically acceptable salts, and solvates thereof, wherein:
m is an Integer from 0 to 3;
p is an integer from 0 to 4;
each R¹ and R² is independently selected from H and C₁-C₆ alkyl;
R³ is -(CR¹R²)₁(4 to 10 membered heterocyclic), wherein t is an integer from 0 to 5, said heterocyclic group is optionally fused to a benzene ring or a C₅-C₈ cycloalkyl group, the -(CR¹R²)ₜ- moiety of the foregoing R³ group optionally includes a carbon-carbon double or triple bond where t is an integer between 2 and 5, and the foregoing R³ groups, including any optional fused rings referred to above, are optionally substituted by 1 to 5 R⁸ groups;
R⁴ is -C≡C-(CR¹⁶R¹⁷)ₜR⁹, -C=C-(CR¹⁶R¹⁷)ₜR⁹, -C≡C-(CR¹⁶R¹⁷)ₖR¹³, or-C=C-(CR¹⁶R¹⁷)ₖR¹³, wherein the attachment point to R⁹ is through a carbon atom of the R⁹ group, each k is an integer from 1 to 3, each t is an integer from 0 to 5, and each m is an integer from 0 to 3;
each R⁵ is independently selected from halo, hydroxy, -NR¹R², C₁-C₆ alkyl, trifluoromethyl, C₁-C₆ alkoxy, trifluoromethoxy, -NR⁶C(O)R¹, -C(O)NR⁶R⁷, - SO₂NR⁶R⁷, -NR⁶C(O)NR⁷R¹, and -NR⁶C(O)OR⁷;
each R⁶, R^{6a} and R⁷ is independently selected from H, C₁-C₆ alkyl, - (CR¹R²)ₜ(C₆-C₁₀aryl), and -(CR¹R²)ₜ(4 to 10 membered heterocyclic), wherein t is an Integer from 0 to 5, 1 or 2 ring carbon atoms of the heterocyclic group are optionally substituted with an oxo (=O) moiety, the alkyl, aryl and heterocyclic moieties of the foregoing R⁶ and R⁷ groups are optionally substituted with 1 to 3 substituents independently selected from halo, cyano, nitro, -NR¹R², trifluoromethyl, trifluoromethoxy, C₁-C₆ alkyl, C₂-C₆ alkenyl, C₂-C₆ alkynyl, hydroxy, and C₁-C₆ alkoxy;
or R⁶ and R⁷, or R^{6a} and R⁷, when attached to a nitrogen atom (including the same nitrogen atom or two separate nitrogen atoms In proximity to each other through interconnection by, for instance, -C(O)- or -SO₂-), can be taken together to form a 4 to 10 membered heterocyclic ring which may Include 1 to 3 additional hetero moieties, in addition to the nitrogen to which said R⁶, R^{6a}, and R⁷ are attached, selected from N, N(R¹), O, and S, provided two O atoms, two S atoms or an O and S atom are not attached directly to each other;
each R⁸ is independently selected from oxo (=O), halo, cyano, nitro, trifluoromethoxy, trifluoromethyl, azido, hydroxy, C₁-C₆ alkoxy, C₁-C₁₀ alkyl, C₂-C₆ alkenyl, C₂-C₆ alkynyl, -C(O)R⁶, -C(O)OR⁶, -OC(O)R⁶, -NR⁶C(O)R⁷, - NR⁶SO₂NR⁷R¹, -NR⁶C(O)NR¹R⁷, -NR⁸C(O)OR⁷, -C(O)NR⁶R⁷, -NR⁶R⁷, -NR⁶OR⁷, -SO₂NR⁶R⁷, -S(O)ⱼ(C₁-C₆ alkyl) wherein j is an integer from 0 to 2, -(CR¹R²)ₜ(C₆-C₁₀ aryl), -(CR¹R²)ₜ(4 to 10 membered heterocyclic), -(CR¹R²)_{q}C(O)(CR¹R²)ₜ(C₆-C₁₀ aryl), -(CR₁R₂)_{q}C(O)(CR¹R²)ₜ(4 to 10 membered heterocyclic), - (CR¹R²)ₜO(CR¹R²)_{q}(C₆-C₁₀ aryl), -(CR¹R²)ₜO(CR¹R²)_{q}(4 to 10 membered heterocyclic), -(CR¹R²)_{q}S(O)ⱼ(CR¹R²)ₜ(C₆-C₁₀ aryl), and -(CR¹R²)_{q}S(O)ⱼ(CR¹R²)ₜ(4 to 10 membered heterocyclic), wherein j is 0, 1 or 2, q and t are each independently an integer from 0 to 5, 1 or 2 ring carbon atoms of the heterocyclic moieties of the foregoing R⁸ groups are optionally substituted with an oxo (=O) moiety, and the alkyl, alkenyl, alkynyl, aryl and heterocyclic moieties of the foregoing R⁸ groups are optionally substituted with 1 to 3 substituents independently selected from halo, cyano, nitro, trifluoromethyl, trifluoromethoxy, azido, -OR⁶, -C(O)R⁶, -C(O)OR⁶, -OC(O)R⁶, -NR⁶C(O)R⁷, -C(O)NR⁶R⁷, -NR⁶R⁷, - NR⁶OR⁷, C₁-C₆ alkyl, C₂-C₆ alkenyl, C₂-C₆ alkynyl, -(CR¹R²)ₜ(C₆-C₁₀ aryl), and - (CR¹R²)ₜ(4 to 10 membered heterocyclic), wherein t is an integer from 0 to 5;
R⁹ is a non-aromatic mono-cyclic ring, a fused or bridged bicyclic ring, or a spirocyclic ring, wherein said ring contains from 3 to 12 carbon atoms in which from 0 to 3 carbon atoms are optionally replaced with a hetero moiety independently selected from N, O, S(O)ⱼ wherein j is an integer from 0 to 2, and - NR¹-, provided that two O atoms, two S(O)ⱼ moieties, an O atom and a S(O)ⱼ moiety, an N atom and an S atom, or an N atom and an O atom are not attached directly to each other within said ring, and wherein the carbon atoms of said ring are optionally substituted with 1 or 2 R⁸ groups;
each R¹¹ Is independently selected from the substituents provided in the definition of R⁸, except R¹¹ is not oxo (=O);
R¹² is R⁶, -OR⁶, -OC(O)R⁶, -OC(O)NR⁶R⁷, -OCO₂R⁶, -S(O)ⱼR⁶, - S(O)ⱼNR⁶R⁷, -NR⁶R⁷, -NR⁶C(O)R⁷, -NR⁶SO₂R⁷, -NR⁶C(O)NR^{6a}R⁷, - NR⁶SO₂NR^{6a}R⁷, -NR⁶CO₂R⁷, CN, -C(O)R⁶, or halo, wherein j is an integer from 0 to 2;
R¹³ is -NR¹R¹⁴ or -OR¹⁴;
R¹⁴ is H, R¹⁵, -C(O)R¹⁵, -SO₂R¹⁵, -C(O)NR¹⁵R⁷, -SO₂NR¹⁵R⁷, or -CO₂R¹⁵;
R¹⁵ is R¹⁸, -(CR¹R²)ₜ(C₆-C₁₀ aryl), -(CR¹R²)ₜ(4 to 10 membered heterocyclic), wherein t is an integer from 0 to 5, 1 or 2 ring carbon atoms of the heterocyclic group are optionally substituted with an oxo (=O) moiety, and the aryl and heterocyclic moieties of the foregoing R¹⁵ groups are optionally substituted with 1 to 3 R⁸ substituents;
each R¹⁶ and R¹⁷ is independently selected from H, C₁-C₆ alkyl, and - CH₂OH, or R¹⁶ and R¹⁷ are taken together as -CH₂CH₂- or -CH₂CH₂CH₂-;
R¹⁶ is C₁-C₆ alkyl wherein each carbon not bound to a N or O atom, or to S(O)ⱼ, wherein j is an integer from 0 to 2, is optionally substituted with R¹²;
and wherein any of the above-mentioned substituents comprising a CH₃ (methyl), CH₂ (methylene), or CH (methine) group, which is not attached to a halogeno, SO or SO₂ group or to a N, O or S atom, is optionally substituted with a group selected from hydroxy, halo, C₁-C₄ alkyl, C₁-C₄ alkoxy and -NR¹R², which comprises reacting a compound of formula 2 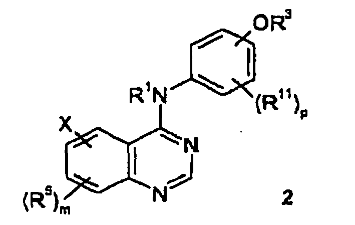 wherein X Is a halide and R¹, R³, R⁵, R¹¹, m and p are as defined for formula 1 with a compound of formula H-C≡C-(CR¹⁶R¹⁷)ₜR⁹, M-C=C-(CR¹⁶R¹⁷)ₜ-R⁹, H-C≡C-(CR¹⁶R¹⁷)ₖR¹³, or M-C=C-(CR¹⁶R¹⁷)ₖR¹³, wherein the attachment point to R⁹ is through a carbon atom of the R⁹ group, each k is an integer from 1 to 3, each t is an integer from 0 to 5, and each m is an integer from 0 to 3, wherein M is selected from the group consisting of H, B(R¹⁹)₂, Al(R²⁰)₂, Sn(R²¹)₃. MgW. or ZnW, wherein R¹⁹ is selected from the group consisting of 9-BBN. C₁-C₁₀ alkyl, C₁-c₁₀ alkoxy, C₃-C₁₀ cycloalkyl, and halo, wherein R²⁰ is selected from the group consisting of C₁-C₁₀ alkyl, C₁-C₁₀ alkoxy, C₃-C₁₀ cycloalkyl, and halo, wherein R²¹ is C₁-C₁₀ alkyl and wherein W is Cl, Br or I, wherein said reaction is carried out in the presence of a palladium catalyst, a ligand, a base, and an optional additive.

2. The method according to claim 1, wherein R³ is -(CR¹R²)ₜ(4 to 10 membered heterocyclic), wherein t is an integer from 0 to 5, and the foregoing R³ groups are optionally substituted by 1 to 3 R⁶ groups.

3. The method according to claim 2, wherein said heterocyclic group is optionally fused to a benzene ring or a C₅-C₈ cycloalkyl group, and the foregoing R³ groups, including any optional fused rings referred to above, are optionally substituted by 1 to 3 R⁸ groups.

4. The method according to claim 1, wherein R³ is selected from 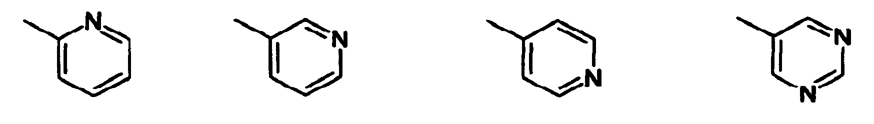 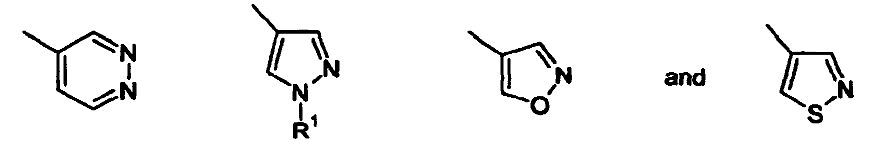 wherein the foregoing R³ groups are optionally substituted by 1 to 3 R⁸ groups.

5. The method according to claim 1, wherein R³ is pyridin-3-yl optionally substituted by 1 to 3 R⁸ groups.

6. The method according to claim 1, wherein R⁴ is -C≡C-(CR¹⁶R¹⁷)ₜR⁹, wherein m is an integer from 0 to 3, and t is an integer from 0 to 5.

7. The method according to claim 1, wherein R⁴ is -C≡C-(CR¹⁶R¹⁷)ₜR⁹, wherein m is an integer from 0 to 3, and t is an integer from 0 to 5, wherein R⁹ is selected from 3-piperidinyl and 4-piperidinyl each of which is optionally substituted with 1 or 2 R⁸ groups.

8. The method according to claim 1 wherein R⁴ is -C=C-(CR¹⁶R¹⁷)ₜ-R⁹, wherein m is an integer from 0 to 3, and t is an integer from 0 to 5.

9. The method according to claim 1, wherein R⁴ is -C=C-(CR¹⁶R¹⁷)ₜR⁹, wherein m is an integer from 0 to 3, and t is an integer from 0 to 5, wherein R⁹ is selected from 3-piperidinyl and 4-piperidinyl (optionally substituted with 1 or 2 R⁸ groups).

10. The method according to claim 1, wherein R⁴ is -C≡C-(CR¹⁶R¹⁷)ₖR¹³, wherein k is an integer from 1 to 3 and m is an integer from 0 to 3.

11. The method according to claim 1, wherein the compound prepared is selected from the group consisting of:
(±)-[3-Methyl-4-(pyridin-3-yloxy)-phenyl]-(6-piperidin-3-ylethynylquinazolin-4-yl)-amine;
2-Methoxy-N-(3-{4-[3-methyl-4-(pyridin-3-yloxy)-phenylamino]-quinazolin-6-yl}-prop-2-ynyl)-acetamide:
(±)-[3-Methyl-4-(6-methyl-pyridin-3-yloxy)-phenyl]-(6-piperidin-3-ylethynyl-quinazolin-4-yl)-amine;
[3-Methyl-4-(6-methyl-pyridin-3-yloxy)-phenyl]-(6-piperidin-4-ylethynyl-quinazoiln-4-yl)-amine;
2-Methoxy-N-(3-{4-[3-methyl-4-(6-methyl-pyridin-3-yloxy)-phenylamino]-quinazolin-6-yl}-prop-2-ynyl)-acetamide;
2-Fluoro-N-(3-{4-[3-methyl-4-(6-methyl-pyridin-3-yloxy)-phenylamino]-quinazolin-6-yl}-prop-2-ynyl)-acetamide;
E-2-Methoxy-N-(3-{4-[3-methyl-4-(6-methyl-pyridin-3-yloxy)-phenylamino]-quinazolin-6-yl}-allyl)-acetamide;
[3-Methyl-4-(pyridin-3-yloxy)-phenyl]-(6-piperidin-4-ylethynyl-quinazolin-4-yl)-amine;
2-Methoxy-N-(1-{4-[3-methyl4-(6-methyl-pyridin-3-yloxy)-phenylamino]-quinazolin-6-ylethynyl}-cyclopropyl)-acetamide;
E-N-(3-{4-[3-Chloro-4-(6-methyl-pyridin-3-yloxy)-phenylamino]-quinazolin-6-yl}-allyl)-2-methoxy-acetamide;
N-(3-{4-[3-Chloro-4-(6-methyl-pyridin-3-yloxy)phenylamino]-quinazolin-6-yl}-prop-2-ynyl)-acetamide;
N-(3-{4-[3-Methyl-4-(6-methyl-pyridin-3-yloxy)-phenylamino]-quinazolin-6-yl}-prop-2-ynyl)-acetamide;
E-N-(3-{4-[3-Chloro-4-(6-methyl-pyridin-3-yloxy)-phenylamino]-quinazolin-6-yl}-allyl)-acetamide;
E-2-Ethoxy-N-(3-{4-[3-methyl-4-(6-methyl-pyridin-3-yloxy)-phenylamino]-quinazolin-6-yl}-allyl)-acetamide;
1-Ethyl-3-(3-{4-[3-methyl-4-(6-methyl-pyridin-3-yloxy)-phenylamino]-quinazolin-6-yl}-prop-2-ynyl)-urea;
Piperazine-1-carboxylic acid (3-{4-[3-methyl-4-(6-methyl-pyridin-3-yloxy)-phenylamino]-quinazolln-6-yl}-prop-2-ynyl)-amide;
(±)-2-Hydroxymethyl-pyrrolidine-1-carboxylic acid (3-{4-[3-methyl-4-(6-methyl-pyrid in-3-yloxy)-phenylamino]-quinazolin-6-yl}-prop-2-ynyl)-amide;
2-Dimethylamino-N-(3-{4-[3-methy)-4-(pyridin-3-yloxy)-phenylamino]-quinazolin-6-yl}-prop-2-ynyl)-acetamide;
E-N-(3-{4-[3-Methyl-4-(6-methyl-pyridln-3-yloxy)-phenylamino]-quinazolin-6-yl}-allyl)-methanesulfonamide;
Isoxazole-5-carboxylic acid (3-{4-[3-methyl-4-(6-methyl-pyridin-3-yloxy)-phenylamino]-quinazolin-6-yl}-prop-2-ynyl)-amide:
1-(1,1-Dimethyl-3-{4-[3-methyl-4-(6-methyl-pyridin-3-yloxy)-phenylamino]-quinazolin-6-yl}-prop-2-ynyl)-3-ethyl-urea;
and the pharmaceutically acceptable salts, and solvates of the foregoing compounds.

## Patentansprüche

1. Verfahren zur Herstellung einer Verbindung der Formel 1 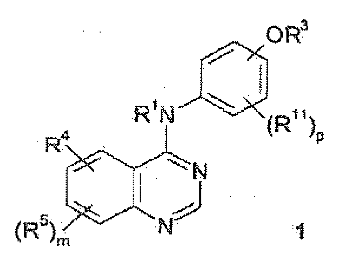 pharmazeutisch unbedenklicher Salze und Solvate davon, wobei:
m für eine ganze Zahl von 0 bis 3 steht;
p für eine ganze Zahl von 0 bis 4 steht;
R¹ und R² jeweils unabhängig voneinander unter H und C₁-C₆-Alkyl ausgewählt sind;
R³ für -(CR¹R²)ₜ(4- bis 10-gliedriger Heterocyclus) steht, wobei t für eine ganze Zahl von 0 bis 5 steht, die Heterocyclusgruppe gegebenenfalls an einen Benzolring oder eine C₅-C₈-Cycloalkylgruppe ankondensiert ist, die - (CR¹R²) ₜ-Gruppierung der obigen Gruppe R³ gegebenenfalls eine Kohlenstoff-Kohlenstoff-Doppelbindung oder -Dreifachbindung enthält, wo t für eine ganze Zahl zwischen 2 und 5 steht, und die obigen Gruppen R³ einschließlich jeglicher oben erwähnter fakultativer ankondensierter Ringe gegebenenfalls durch 1 bis 5 Gruppen R⁸ substituiert sind;
R⁴ für -C≡C-(CR¹⁶R¹⁷) ₜR⁹, -C≡C-(CR¹⁶R¹⁷)ₜR⁹, -C≡C-(CR¹⁶R¹⁷)ₖR¹³ oder -C=C-(CR¹⁶R¹⁷)ₖR¹³ steht, wobei die Verknüpfung mit R⁹ über ein Kohlenstoffatom der Gruppe R⁹ erfolgt, k jeweils für eine ganze Zahl von 1 bis 3 steht, t jeweils für eine ganze Zahl von 0 bis 5 steht und m jeweils für eine ganze Zahl von 0 bis 3 steht;
R⁵ jeweils unabhängig voneinander unter Halogen, Hydroxy, -NR¹R², C₁-C₆-Alkyl, Trifluormethyl, C₁-C₆-Alkoxy, Trifluormethoxy, -NR⁶C(O)R¹, -C(O)NR⁶R⁷, -SO₂NR⁶R⁷, -NR⁶C (O) NR⁷R¹ und -NR⁶C (O)OR⁷ ausgewählt ist;
R⁶, R^{6a} und R⁷ jeweils unabhängig voneinander unter H, C₁-C₆-Alkyl, - (CR¹R²)ₜ(C₆-C₁₀-Aryl) und -(CR¹R²)ₜ(4- bis 10-gliedriger Heterocyclus) ausgewählt sind, wobei t für eine ganze Zahl von 0 bis 5 steht, 1 oder 2 Ringkohlenstoffatome der Heterocyclusgruppe gegebenenfalls durch eine Oxogruppierung (=0) substituiert sind, die Alkyl-, Aryl- und Heterocyclusgruppierungen der obigen Gruppen R⁶ und R⁷ gegebenenfalls durch 1 bis 3 unabhängig voneinander unter Halogen, Cyano, Nitro, -NR¹R², Trifluormethyl, Trifluormethoxy, C₁-C₆-Alkyl, C₂-C₆-Alkenyl, C₂-C₆-Alkinyl, Hydroxy und C₁-C₆-Alkoxy ausgewählte Substituenten substituiert sind;
oder R⁶ und R⁷ oder R^{6a} und R⁷, wenn sie an ein Stickstoffatom (einschließlich desselben Stickstoffatoms oder zweier separater Stickstoffatome, die einander durch Verknüpfung durch beispielsweise -C(O)- oder -SO₂- nahe stehen) gebunden sind, gemeinsam einen 4- bis 10-gliedrigen heterocyclischen Ring bilden können, welcher neben dem Stickstoff, an den R⁶, R^{6a} und R⁷ gebunden sind, 1 bis 3 zusätzliche, unter N, N(R¹), O und S ausgewählte Heterogruppierungen enthalten kann, mit der Maßgabe, daß zwei O-Atome, zwei S-Atome oder ein 0- und ein S-Atom nicht direkt aneinander gebunden sind;
R⁸ jeweils unabhängig voneinander unter Oxo (=O), Halogen, Cyano, Nitro, Trifluormethoxy, Trifluormethyl, Azido, Hydroxy, C₁-C₆-Alkoxy, C₁-C₁₀-Alkyl, C₂-C₆-Alkenyl, C₂-C₆-Alkinyl, -C(O)R⁶, -C(O)OR⁶, -OC(O)R⁶, -NR⁶C (0) R⁷, -NR⁶SO₂NR⁷R¹, -NR⁶C (O) NR⁷R¹, -NR⁶C (0) OR⁷, -C (0) NR⁶R⁷, -NR⁶R⁷, -NR⁶OR⁷, SO₂NR⁶R⁷, -S(O)ⱼ(C₁-C₆-Alkyl), worin j für eine ganze Zahl von 0 bis 2 steht, -(CR¹R²)ₜ(C₆-C₁₀-Aryl), - (CR¹R²) ₜ (4- bis 10-gliedriger Heterocyclus), -(CR¹R²)_{q}C (O) (CR¹R²)ₜ(C₆-C₁₀-Aryl) ,
-(CR¹R²)_{q}C(O) (CR¹R²)ₜ(4- bis 10-gliedriger Heterocyclus), -(CR¹R²)ₜO (CR¹R²)_{q} (C₆-C₁₀-Aryl), - (CR¹R²)ₜO(CR¹R²)_{q}(4- bis 10-gliedriger Heterocyclus), - (CR¹R²)_{q}S(O)ⱼ(CR¹R²)ₜ(C₆-C₁₀-Aryl) und - (CR¹R²)_{q}S(O)ⱼ(CR¹R²)ₜ (4- bis 10-gliedriger Heterocyclus) ausgewählt sind, wobei j für 0, 1 oder 2 steht, q und t jeweils unabhängig voneinander für eine ganze Zahl von 0 bis 5 stehen, 1 oder 2 Ringkohlenstoffatome der Heterocyclusgruppierungen der obigen Gruppen R⁸ gegebenenfalls durch eine Oxogruppierung (=0) substituiert sind und die Alkyl-, Alkenyl-, Alkinyl-, Aryl- und Heterocyclusgruppierungen der obigen Gruppen R⁸ gegebenenfalls durch 1 bis 3 unabhängig voneinander unter Halogen, Cyano, Nitro, Trifluormethyl, Trifluormethoxy, Azido, -OR⁶, -C(O)R⁶, -C(O)OR⁶, -OC(O)R⁶, -NR⁶C (O) R⁷, -C (O) NR⁶R⁷, -NR⁶R⁷, -NR⁶OR⁷, C₁-C₆-Alkyl, C₂-C₆-Alkenyl, C₂-C₆-Alkinyl, - (CR¹R²)ₜ(C₆-C₁₀-Aryl) und - (CR¹R²)ₜ(4- bis 10-gliedriger Heterocyclus) ausgewählte Substituenten substituiert sind, wobei t für eine ganze Zahl von 0 bis 5 steht;
R⁹ für einen nichtaromatischen monocyclischen Ring, einen kondensierten oder verbrückten bicyclischen Ring oder einen spirocyclischen Ring steht, wobei der Ring 3 bis 12 Kohlenstoffatome enthält, worin 0 bis 3 Kohlenstoffatome gegebenenfalls durch eine unter N, 0, S(O)ⱼ, worin j für eine ganze Zahl von 0 bis 2 steht, und -NR¹- ausgewählte Heterogruppierung ersetzt sind, mit der Maßgabe, daß zwei O-Atome, zwei S(O)ⱼ-Gruppierungen, ein O-Atom und eine S(O)ⱼ-Gruppierung, ein N-Atom und ein S-Atom oder ein N-Atom und ein O-Atom in dem Ring nicht direkt aneinander gebunden sind, und wobei die Kohlenstoffatome des Rings gegebenenfalls durch 1 oder 2 Gruppen R⁸ substituiert sind;
R¹¹ jeweils unabhängig voneinander unter den bei der Definition von R⁸ aufgeführten Substituenten ausgewählt ist, außer daß R¹¹ nicht für Oxo (=O) steht;
R¹² für R⁶, -OR⁶, -OC(O)R⁶, -OC(O)NR⁶R⁷, -OCO₂R⁶, -S(O)ⱼR⁶, -S(O)ⱼNR⁶R⁷, -NR⁶R⁷, -NR⁶C (O) R⁷, -NR⁶SO₂R⁷, -NR⁶C(O)NR^{6a}R⁷, -NR⁶SO₂NR^{6a}R⁷, -NR⁶CO₂R⁷, CN, -C (O) R⁶ oder Halogen steht, wobei j für eine ganze Zahl von 0 bis 2 steht;
R¹³ für -NR¹R¹⁴ oder -OR¹⁴ steht;
R¹⁴ für H, R¹⁵, -C(O)R¹⁵, -SO₂R¹⁵, -C (O)NR¹⁵R⁷, -SO₂NR¹⁵R⁷ oder -CO₂R¹⁵ steht;
R¹⁵ für R¹⁸, -(CR¹R²)ₜ(C₆-C₁₀-Aryl), -(CR¹R²)ₜ(4- bis 10-gliedriger Heterocyclus) steht, wobei t für eine ganze Zahl von 0 bis 5 steht, 1 oder 2 Ringkohlenstoffatome der Heterocyclusgruppe gegebenenfalls durch eine Oxogruppierung (=0) substituiert sind und die Aryl- und Heterocyclusgruppierungen der obigen Gruppen R¹⁵ gegebenenfalls durch 1 bis 3 Substituenten R⁸ substituiert sind;
R¹⁶ und R¹⁷ jeweils unabhängig voneinander unter H, C₁-C₆-Alkyl und -CH₂OH ausgewählt sind oder R¹⁶ und R¹⁷ gemeinsam für -CH₂CH₂- oder -CH₂CH₂CH₂- stehen;
R¹⁸ für C₁-C₆-Alkyl steht, wobei jeder nicht an ein N- oder O-Atom oder an -S(O)ⱼ, worin j für eine ganze Zahl von 0 bis 2 steht, gebundene Kohlenstoff gegebenenfalls durch R¹² substituiert ist;
und wobei jeder der oben aufgeführten Substituenten, der eine CH₃-Gruppe (Methylgruppe), eine CH₂-Gruppe (Methylengruppe) oder eine CH-Gruppe (Methingruppe) umfaßt, die nicht an eine Halogen-, SO- oder SO₂-Gruppe oder ein N-, 0- oder S-Atom gebunden ist, gegebenenfalls durch eine unter Hydroxy, Halogen, C₁-C₄-Alkyl, C₁-C₄-Alkoxy und -NR¹R² ausgewählte Gruppe substituiert ist, bei dem man eine Verbindung der Formel 2 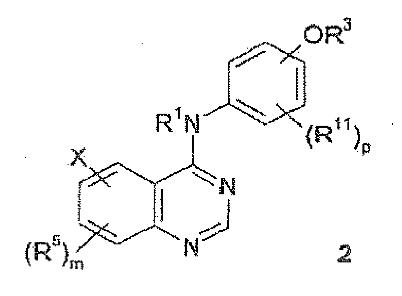
worin X für ein Halogenid steht und R¹, R³, R⁵, R¹¹, m und p die für Formel 1 angegebene Bedeutung besitzen, mit einer Verbindung der Formel H-C≡C-(CR¹⁶R¹⁷)ₜR⁹, M-C≡C- (CR¹⁶R¹⁷)ₜR⁹, H-C≡C-(CR¹⁶R¹⁷) ₖR¹³ oder M-C=C-(CR¹⁶R¹⁷)ₖR¹³, wobei die Verknüpfung mit R⁹ über ein Kohlenstoffatom der Gruppe R⁹ erfolgt, k jeweils für eine ganze Zahl von 1 bis 3 steht, t jeweils für eine ganze Zahl von 0 bis 5 steht und m jeweils für eine ganze Zahl von 0 bis 3 steht, wobei M aus der Gruppe bestehend aus H, B(R¹⁹)₂, Al(R²⁰)₂, Sn(R²¹)₃, MgW oder ZnW ausgewählt ist, wobei R¹⁹ aus der Gruppe bestehend aus 9-BBN, C₁-C₁₀-Alkyl, C₁-C₁₀-Alkoxy, C₃-C₁₀-Cycloalkyl und Halogen ausgewählt ist, wobei R²⁰ aus der Gruppe bestehend aus C₁-C₁₀-Alkyl, C₁-C₁₀-Alkoxy, C₃-C₁₀-Cycloalkyl und Halogen ausgewählt ist, wobei R²¹ für C₁-C₁₀-Alkyl steht und W für Cl, Br oder I steht, in Gegenwart eines Palladiumkatalysators, eines Liganden, einer Base und eines fakultativen Additivs umsetzt.

2. Verfahren nach Anspruch 1, bei dem R³ für -(CR¹R²)ₜ (4- bis 10-gliedriger Heterocyclus) steht, wobei t für eine ganze Zahl von 0 bis 5 steht, und die obigen Gruppen R³ gegebenenfalls durch 1 bis 3 Gruppen R⁸ substituiert sind.

3. Verfahren nach Anspruch 2, bei dem die Heterocyclusgruppe gegebenenfalls an einen Benzolring oder eine C₅-C₈-Cycloalkylgruppe ankondensiert ist und die obigen Gruppen R³ einschließlich jeglicher oben erwähnter fakultativer ankondensierter Ringe gegebenenfalls durch 1 bis 3 Gruppen R⁸ substituiert sind.

4. Verfahren nach Anspruch 1, bei dem R³ unter 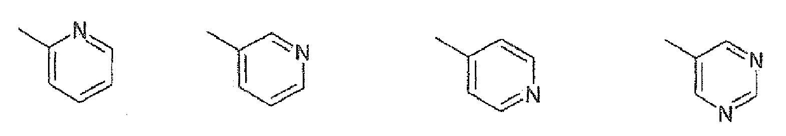 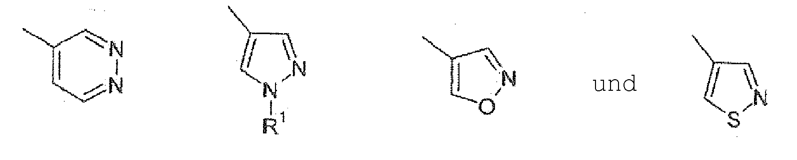 ausgewählt ist, wobei die obigen Gruppen R³ gegebenenfalls durch 1 bis 3 Gruppen R⁸ substituiert sind.

5. Verfahren nach Anspruch 1, bei dem R³ für Pyridin-3-yl, das gegebenenfalls durch 1 bis 3 Gruppen R⁸ substituiert ist, steht.

6. Verfahren nach Anspruch 1, bei dem R⁴ für -C≡C-(CR¹⁶R¹⁷)ₜR⁹ steht, wobei m für eine ganze Zahl von 0 bis 3 steht und t für eine ganze Zahl von 0 bis 5 steht.

7. Verfahren nach Anspruch 1, bei dem R⁴ für -C≡C-(CR¹⁶R¹⁷)ₜR⁹ steht, wobei m für eine ganze Zahl von 0 bis 3 steht und t für eine ganze Zahl von 0 bis 5 steht, wobei R⁹ unter 3-Piperidinyl und 4-Piperidinyl, jeweils gegebenenfalls substituiert durch 1 oder 2 Gruppen R⁸, ausgewählt ist.

8. Verfahren nach Anspruch 1, bei dem R⁴ für -C=C-(CR¹⁶R¹⁷)ₜR⁹ steht, wobei m für eine ganze Zahl von 0 bis 3 steht und t für eine ganze Zahl von 0 bis 5 steht.

9. Verfahren nach Anspruch 1, bei dem R⁴ für -C=C-(CR¹⁶R¹⁷)ₜR⁹ steht, wobei m für eine ganze Zahl von 0 bis 3 steht und t für eine ganze Zahl von 0 bis 5 steht, wobei R⁹ unter 3-Piperidinyl und 4-Piperidinyl (gegebenenfalls substituiert durch 1 oder 2 Gruppen R⁸) ausgewählt ist.

10. Verfahren nach Anspruch 1, bei dem R⁴ für -C≡C-(CR¹⁶R¹⁷)ₖR¹³ steht, wobei k für eine ganze Zahl von 1 bis 3 steht und m für eine ganze Zahl von 0 bis 3 steht.

11. Verfahren nach Anspruch 1, bei dem die hergestellte Verbindung aus der Gruppe bestehend aus:
(±)-[3-Methyl-4-(pyridin-3-yloxy)phenyl]-(6-piperidin-3-ylethinylchinazolin-4-yl)amin;
2-Methoxy-N-(3-{4-[3-methyl-4-(pyridin-3-yloxy)-phenylamino]chinazolin-6-yl}prop-2-inyl)acetamid;
(±)-[3-Methyl-4-(6-methylpyridin-3-yloxy)phenyl]-(6-piperidin-3-ylethinylchinazolin-4-yl)amin;
[3-Methyl-4-(6-methylpyridin-3-yloxy)phenyl]-(6-piperidin-4-ylethinylchinazolin-4-yl)amin;
2-Methoxy-N-(3-{4-[3-methyl-4-(6-methylpyridin-3-yloxy)phenylamino]chinazolin-6-yl}prop-2-inyl)-acetamid;
2-Fluor-N-(3-{4-[3-methyl-4-(6-methylpyridin-3-yloxy)phenylamino]chinazolin-6-yl}prop-2-inyl)-acetamid;
E-2-Methoxy-N-(3-{4-[3-methyl-4-(6-methylpyridin-3-yloxy)phenylamino]chinazolin-6-yl}allyl)-acetamid;
[3-Methyl-4-(pyridin-3-yloxy)phenyl]-(6-piperidin-4-ylethinylchinazolin-4-yl)amin;
2-Methoxy-N-(1-{4-[3-methyl-4-(6-methylpyridin-3-yloxy)phenylamino]chinazolin-6-ylethinyl}cyclopropyl)acetamid;
E-N-(3-{4-[3-Chlor-4-(6-methylpyridin-3-yloxy)-phenylamino]chinazolin-6-yl}allyl)-2-methoxy-acetamid;
N-(3-{4-[3-Chlor-4-(6-methylpyridin-3-yloxy)-phenylamino]chinazolin-6-yl}prop-2-inyl)acetamid;
N-(3-{4-[3-Methyl-4-(6-methylpyridin-3-yloxy)-phenylamino]chinazolin-6-yl}prop-2-inyl)acetamid;
E-N-(3-{4-[3-Chlor-4-(6-methylpyridin-3-yloxy)-phenylamino]chinazolin-6-yl}allyl)acetamid;
E-2-Ethoxy-N-(3-{4-[3-methyl-4-(6-methylpyridin-3-yloxy)phenylamino]chinazolin-6-yl}allyl)acetamid;
1-Ethyl-3-(3-{4-[3-methyl-4-(6-methylpyridin-3-yloxy)phenylamino]chinazolin-6-yl}prop-2-inyl)-harnstoff;
Piperazin-1-carbonsäure-(3-{4-[3-methyl-4-(6-methylpyridin-3-yloxy)phenylamino]chinazolin-6-yl}prop-2-inyl)amid;
(±)-2-Hydroxymethylpyrrolidin-1-carbonsäure-(3-{4-[3-methyl-4-(6-methylpyridin-3-yloxy)phenylamino]-chinazolin-6-yl}prop-2-inyl)amid;
2-Dimethylamino-N-(3-{4-[3-methyl-4-(pyridin-3-yl-oxy)phenylamino]chinazolin-6-yl}prop-2-inyl)-acetamid;
E-N-(3-{4-[3-Methyl-4-(6-methylpyridin-3-yloxy)-phenylamino]chinazolin-6-yl}allyl)methansulfonamid;
Isoxazol-5-carbonsäure-(3-{4-[3-methyl-4-(6-methylpyridin-3-yloxy)phenylamino]chinazolin-6-yl}prop-2-inyl)amid;
1-(1,1-Dimethyl-3-{4-[3-methyl-4-(6-methylpyridin-3-yloxy)phenylamino]chinazolin-6-yl}prop-2-inyl)-3-ethylharnstoff
und den pharmazeutisch unbedenklichen Salzen und Solvaten der obigen Verbindungen ausgewählt wird.

## Revendications

1. Procédé de préparation d'un composé de formule 1 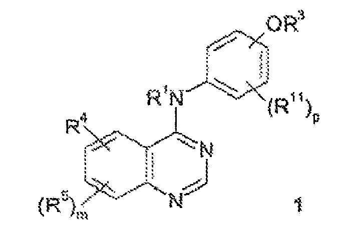 des sels pharmaceutiquement acceptables et des solvates de celui-ci, dans laquelle :
m est un entier de 0 à 3 ;
p est un entier de 0 à 4 ;
chaque R¹ et R² est choisi indépendamment parmi H et alkyle en C₁-C₆ ;
R³ est - (CR¹R²)ₜ(hétérocycle de 4 à 10 chaînons), où t est un entier de 0 à 5, ledit groupement hétérocyclique est éventuellement condensé sur un cycle benzène ou un groupement cycloalkyle en C₅-C₈, le motif - (CR¹R²)ₜ- du groupement R³ précédent comporte éventuellement une double ou triple liaison carbone-carbone où t est un entier compris entre 2 et 5, et les groupements R³ précédents, y compris tous les cycles condensés éventuels auxquels il est fait référence ci-dessus, sont éventuellement substitués par 1 à 5 groupements R⁸ ;
R⁴ est -C≡C- (CR¹⁶R¹⁷)ₜR⁹, -C=C-(CR¹⁶R¹⁷)ₜR⁹, -C≡C-(CR¹⁶R¹⁷)ₖR¹³ ou -C=C- (CR¹⁶R¹⁷)ₖR¹³, où le point d'attachement à R⁹ est par un atome de carbone du groupement R⁹, chaque k est un entier de 1 à 3, chaque t est un entier de 0 à 5 et chaque m est un entier de 0 à 3 ;
chaque R⁵ est choisi indépendamment parmi halogéno, hydroxy, -NR¹R², alkyle en C₁-C₆, trifluorométhyle, alcoxy en C₁-C₆, trifluorométhoxy, -NR⁶C(O)R¹, - C(O)NR⁶R⁷, -SO₂NR⁶R⁷, -NR⁶C (O) NR⁷R¹ et -NR⁶C (O) OR⁷ ;
chaque R⁶, R^{6a} et R⁷ est choisi indépendamment parmi H, alkyle en C₁-C₆, -(CR¹R²)ₜ(aryle en C₆-C₁₀) et -(CR¹R²)ₜ(hétérocycle de 4 à 10 chaînons), où t est un entier de 0 à 5, 1 ou 2 atomes de carbone du cycle du groupement hétérocyclique sont éventuellement substitués par un motif oxo (=O), les motifs alkyle, aryle et hétérocycliques des groupements R⁶ et R⁷ précédents sont éventuellement substitués par 1 à 3 substituants choisis indépendamment parmi halogéno, cyano, nitro, - NR¹R², trifluorométhyle, trifluorométhoxy, alkyle en C₁-C₆, alcényle en C₂-C₆, alcynyle en C₂-C₆, hydroxy et alcoxy en C₁-C₆ ;
ou R⁶ et R⁷, ou R^{6a} et R⁷, lorsqu'ils sont liés à un atome d'azote (y compris le même atome d'azote ou deux atomes d'azote distincts à proximité l'un de l'autre grâce à l'interconnexion, par exemple, par -C(O)- ou -SO₂-) , peuvent être pris ensemble pour former un cycle hétérocyclique de 4 à 10 chaînons qui peut comporter 1 à 3 motifs hétéro supplémentaires, outre l'azote auquel lesdits R⁶, R^{6a} et R⁷ sont liés, choisis parmi N, N (R¹), O et S, à condition que deux atomes d'O, deux atomes de S ou un atome d'O et de S ne soient pas liés directement l'un à l'autre ;
chaque R⁸ est choisi indépendamment parmi oxo (=O), halogéno, cyano, nitro, trifluorométhoxy, trifluorométhyle, azido, hydroxy, alcoxy en C₁-C₆, alkyle en C₁-C₁₀, alcényle en C₂-C₆, alcynyle en C₂-C₆, -C (O) R⁶, -C (O) OR⁶, -OC (O) R⁶, -NR⁶C (O) R⁷, - NR⁶ SO₂NR⁷R¹, -NR⁶C (0) NR¹R⁷, -NR⁶C(O) OR⁷, -C(O) NR⁶R⁷, - NR⁶R⁷, -NR⁶OR⁷, -SO₂NR⁶R⁷, -S(O)ⱼ(alkyle en C₁-C₆), où j est un entier de 0 à 2, - (CR¹R²)ₜ (aryle en C₆-C₁₀), -(CR¹R²)ₜ(hétérocycle de 4 à 10 chaînons), -(CR¹R²)_{q}C (O) (CR¹R²)ₜ(aryle en C₆-C₁₀), -(CR¹R²)_{q}C (O) (CR¹R²)ₜ(hétérocycle de 4 à 10 chaînons) , -(CR¹R²)ₜO (CR¹R²)_{q}(aryle en C₆-C₁₀), -(CR¹R²)ₜO(CR¹R²)_{q}(hétérocycle de 4 à 10 chaînons), -(CR¹R²)_{q}S(O)ⱼ(CR¹R²)ₜ(aryle en C₆-C₁₀) et -(CR¹R²)_{q}S(O)ⱼ(CR¹R²)ₜ(hétérocycle de 4 à 10 chaînons), où j est 0, 1 ou 2, q et t sont chacun indépendamment un entier de 0 à 5, 1 ou 2 atomes de carbone du cycle des motifs hétérocycliques des groupements R⁸ précédents sont éventuellement substitués par un motif oxo (=O), et les motifs alkyle, alcényle, alcynyle, aryle et hétérocycliques des groupements R⁸ précédents sont éventuellement substitués par 1 à 3 substituants choisis indépendamment parmi halogéno, cyano, nitro, trifluorométhyle, trifluorométhoxy, azido, -OR⁶, -C(O)R⁶, -C(O)OR⁶, -OC(O)R⁶, -NR⁶C(O)R⁷, -C(O)NR⁶R⁷, -NR⁶R⁷, -NR⁶OR⁷, alkyle en C₁-C₆, alcényle en C₂-C₆, alcynyle en C₂-C₆, -(CR¹R²)ₜ (aryle en C₆-C₁₀) et - (CR¹R²)ₜ (hétérocycle de 4 à 10 chaînons), où t est un entier de 0 à 5 ;
R⁹ est un cycle monocyclique non-aromatique, un cycle bicyclique condensé ou ponté, ou un cycle spirocyclique, où ledit cycle contient de 3 à 12 atomes de carbone parmi lesquels de 0 à 3 atomes de carbone sont éventuellement remplacés par un motif hétéro choisi indépendamment parmi N, 0, S(O)ⱼ, où j est un entier de 0 à 2, et -NR¹-, à condition que deux atomes d'O, deux motifs S(O)ⱼ, un atome d'O et un motif S(O)ⱼ, un atome de N et un atome de S, ou un atome de N et un atome d'O ne soient pas liés directement l'un à l'autre dans ledit cycle, et où les atomes de carbone dudit cycle sont éventuellement substitués par 1 ou 2 groupements R⁸ ;
chaque R¹¹ est choisi indépendamment parmi les substituants donnés dans la définition de R⁸, sauf que R¹¹ n'est pas oxo (=O) ;
R¹² est R⁶, -OR⁶, -OC(O)R⁶, -OC(O)NR⁶R⁷, -OCO₂R⁶, -S(O)ⱼR⁶, -S(O)ⱼNR⁶R⁷, -NR⁶R⁷, -NR⁶C (O) R⁷, -NR⁶SO₂R⁷, -NR⁶C(O)NR^{6a}R⁷, -NR⁶SO₂NR^{6a}R⁷, -NR⁶CO₂R⁷, CN, -C (O) R⁶ ou halogéno, où j est un entier de 0 à 2 ;
R¹³ est -NR¹R¹⁴ ou -OR¹⁴ ;
R¹⁴ est H, R¹⁵, -C(O)R¹⁵, -SO₂R¹⁵, -C (O)NR¹⁵R⁷, -SO₂NR¹⁵R⁷ ou -CO₂R¹⁵;
R¹⁵ est R¹⁸, -(CR¹R²)ₜ (aryle en C₆-C₁₀), - (CR¹R²)ₜ (hétérocycle de 4 à 10 chaînons), où t est un entier de 0 à 5, 1 ou 2 atomes de carbone du cycle du groupement hétérocyclique sont éventuellement substitués par un motif oxo (=O), et les motifs aryle et hétérocycliques des groupements R¹⁵ précédents sont éventuellement substitués par 1 à 3 substituants R⁸ ;
chaque R¹⁶ et R¹⁷ est choisi indépendamment parmi H, alkyle en C₁-C₆ et -CH₂OH, ou R¹⁶ et R¹⁷ sont pris ensemble comme -CH₂CH₂- ou -CH₂CH₂CH₂- ;
R¹⁸ est alkyle en C₁-₆, où chaque carbone non lié à un atome de N ou de 0, ou à -S(O)ⱼ, où j est un entier de 0 à 2, est éventuellement substitué par R¹²;
et où l'un quelconque des substituants mentionnés ci-dessus comprenant un groupement CH₃ (méthyle), CH₂ (méthylène) ou CH (méthine), qui n'est pas lié à un groupement halogéno, SO ou SO₂, ou à un atome de N, d'O ou de S, est éventuellement substitué par un groupement choisi parmi hydroxy, halogéno, alkyle en C₁-C₄, alcoxy en C₁-C₄ et -NR¹R²-, qui comprend la réaction d'un composé de formule 2 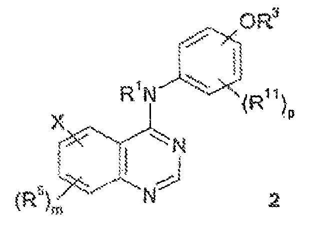
dans laquelle X est un halogénure et R¹, R³, R⁵, R¹¹, m et p sont tels que définis pour la formule 1, avec un composé de formule H-C≡C-(CR¹⁶R¹⁷)ₜR⁹, M-C=C-(CR¹⁶R¹⁷)ₜR⁹, H-C≡C-(CR¹⁶R¹⁷)ₖR¹³ ou M-C=C-(CR¹⁶R¹⁷)ₖR¹³, où le point d'attachement à R⁹ est par un atome de carbone du groupement R⁹, chaque k est un entier de 1 à 3, chaque t est un entier de 0 à 5 et chaque m est un entier de 0 à 3, où M est choisi dans le groupe constitué de H, B(R¹⁹)₂, Al(R²⁰)₂, Sn (R²¹) ₃, MgW ou ZnW, où R¹⁹ est choisi dans le groupe constitué de 9-BBN, alkyle en C₁-C₁₀, alcoxy en C₁-C₁₀, cycloalkyle en C₃-C₁₀ et halogéno, où R²⁰ est choisi dans le groupe constitué d'alkyle en C₁-C₁₀, alcoxy en C₁-C₁₀, cycloalkyle en C₃-C₁₀ et halogéno, où R²¹ est alkyle en C₁-C₁₀ et W est Cl, Br ou I, où ladite réaction est effectuée en présence d'un catalyseur au palladium, d'un ligand, d'une base et d'un additif éventuel.

2. Procédé selon la revendication 1, **caractérisé en ce que** R³ est - (CR¹R²)ₜ (hétérocycle de 4 à 10 chaînons), où t est un entier de 0 à 5, et les groupements R³ précédents sont éventuellement substitués par 1 à 3 groupements R⁸.

3. Procédé selon la revendication 2, **caractérisé en ce que** ledit groupement hétérocyclique est éventuellement condensé sur un cycle benzène ou un groupement cycloalkyle en C₅-C₈, et les groupements R³ précédents, y compris tous les cycles condensés éventuels auxquels il est fait référence ci-dessus, sont éventuellement substitués par 1 à 3 groupements R⁸.

4. Procédé selon la revendication 1, **caractérisé en ce que** R³ est choisi parmi : 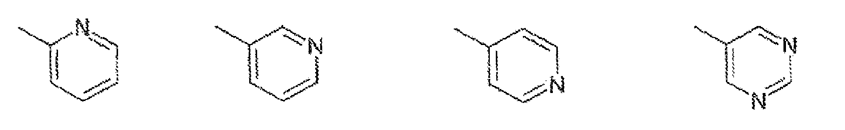 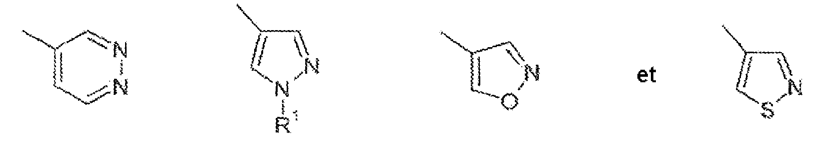 où les groupements R³ précédents sont éventuellement substitués par 1 à 3 groupements R⁸.

5. Procédé selon la revendication 1, **caractérisé en ce que** R³ est pyridin-3-yle éventuellement substitué par 1 à 3 groupements R⁸.

6. Procédé selon la revendication 1, **caractérisé en ce que** R⁴ est -C≡C-(CR¹⁶R¹⁷)ₜR⁹, où m est un entier de 0 à 3 et t est un entier de 0 à 5.

7. Procédé selon la revendication 1, **caractérisé en ce que** R⁴ est -C≡C-(CR¹⁶R¹⁷)ₜR⁹, où m est un entier de 0 à 3 et t est un entier de 0 à 5, où R⁹ est choisi parmi 3-pipéridinyle et 4-pipéridinyle, chacun d'eux étant éventuellement substitué par 1 ou 2 groupements R⁸.

8. Procédé selon la revendication 1, **caractérisé en ce que** R⁴ est -C=C-(CR¹⁶R¹⁷)ₜR⁹, où m est un entier de 0 à 3 et t est un entier de 0 à 5 .

9. Procédé selon la revendication 1, **caractérisé en ce que** R⁴ est -C=C-(CR¹⁶R¹⁷)ₜR⁹, où m est un entier de 0 à 3 et t est un entier de 0 à 5, où R⁹ est choisi parmi 3-pipéridinyle et 4-pipéridinyle (éventuellement substitué par 1 ou 2 groupements R⁸).

10. Procédé selon la revendication 1, **caractérisé en ce que** R⁴ est -C≡C-(CR¹⁶R¹⁷)ₖR¹³, où k est un entier de 1 à 3 et m est un entier de 0 à 3.

11. Procédé selon la revendication 1, **caractérisé en ce que** le composé préparé est choisi dans le groupe constitué de :
(+)-[3-méthyl-4-(pyridin-3-yloxy)-phényl]-(6-pipéridin-3-yléthynylquinazolin-4-yl)-amine ;
2-méthoxy-N-(3-{4-[3-méthyl-4-(pyridin-3-yloxy)-phénylamino]-quinazolin-6-yl}-prop-2-ynyl)-acétamide ;
(±)-[3-méthyl-4-(6-méthylpyridin-3-yloxy)-phényl]-(6-pipéridin-3-yléthynylquinazolin-4-yl)-amine ;
[3-méthyl-4-(6-méthylpyridin-3-yloxy)-phényl]-(6-pipéridin-4-yléthynylquinazolin-4-yl)-amine ;
2-méthoxy-N-(3-{4-[3-méthyl-4-(6-méthylpyridin-3-yloxy)-phénylamino]-quinazolin-6-yl}-prop-2-ynyl)-acétamide ;
2-fluoro-N-(3-{4-[3-méthyl-4-(6-méthylpyridin-3-yloxy)-phénylamino]-quinazolin-6-yl}-prop-2-ynyl)-acétamide ;
E-2-méthoxy-N-(3-{4-[3-méthyl-4-(6-méthylpyridin-3-yloxy)-phénylamino]-quinazolin-6-yl}-allyl)-acétamide ;
[3-méthyl-4-(pyridin-3-yloxy)-phényl]-(6-pipéridin-4-yléthynylquinazolin-4-yl)-amine ;
2-méthoxy-N-(1-{4-[3-méthyl-4-(6-méthylpyridin-3-yloxy)-phénylamino]-quinazolin-6-yléthynyl}-cyclopropyl)-acétamide ;
E-N-(3-{4-[3-chloro-4-(6-méthylpyridin-3-yloxy)-phénylamino]-quinazolin-6-yl}-allyl)-2-méthoxy-acétamide ;
N-(3-{4-[3-chloro-4-(6-méthylpyridin-3-yloxy)-phénylamino]-quinazolin-6-yl}-prop-2-ynyl)-acétamide ;
N-(3-{4-[3-méthyl-4-(6-méthylpyridin-3-yloxy)-phénylamino]-quinazolin-6-yl}-prop-2-ynyl)-acétamide ;
E-N-(3-{4-[3-chloro-4-(6-méthylpyridin-3-yloxy)-phénylamino]-quinazolin-6-yl}-allyl)-acétamide ;
E-2-éthoxy-N-(3-{4-[3-méthyl-4-(6-méthylpyridin-3-yloxy)-phénylamino]-quinazolin-6-yl}-allyl)-acétamide ;
1-éthyl-3-(3-{4-[3-méthyl-4-(6-méthylpyridin-3-yloxy)-phénylamino]-quinazolin-6-yl}-prop-2-ynyl)-urée ;
(3-{4-[3-méthyl-4-(6-méthylpyridin-3-yloxy)-phénylamino]-quinazolin-6-yl}-prop-2-ynyl)-amide d'acide pipérazine-1-carboxylique ;
(3-{4-[3-méthyl-4-(6-méthylpyridin-3-yloxy)-phénylamino]-quinazolin-6-yl}-prop-2-ynyl)-amide d'acide (±)-2-hydroxyméthylpyrrolidine-1-carboxylique ;
2-diméthylamino-N-(3-{4-[3-méthyl-4-(pyridin-3-yl-oxy)-phénylamino]-quinazolin-6-yl}-prop-2-ynyl)-acétamide ;
E-N-(3-{4-[3-méthyl-4-(6-méthylpyridin-3-yloxy)-phénylamino]-quinazolin-6-yl}-allyl)-méthane-sulfonamide ;
(3-{4-[3-méthyl-4-(6-méthylpyridin-3-yloxy)-phénylamino]-quinazolin-6-yl}-prop-2-ynyl)-amide d'acide d'isoxazole-5-carboxylique ;
1-(1,1-diméthyl-3-{4-[3-méthyl-4-(6-méthylpyridin-3-yloxy)-phénylamino]-quinazolin-6-yl}-prop-2-ynyl)-3-éthylurée ;
et les sels pharmaceutiquement acceptables et les solvates de ces composés.
